(19) **Europäisches Patentamt / European Patent Office / Office européen des brevets**

(11) **EP 2 905 363 B1**

(12) **EUROPEAN PATENT SPECIFICATION**

(45) Date of publication and mention
of the grant of the patent:
**19.02.2020 Bulletin 2020/08**

(21) Application number: **13844515.0**

(22) Date of filing: **27.09.2013**

(51) Int Cl.:
**G16B 5/00** (2019.01)

(86) International application number:
**PCT/JP2013/076248**

(87) International publication number:
**WO 2014/054526 (10.04.2014 Gazette 2014/15)**

(54) **APPROVAL PREDICTION DEVICE, APPROVAL PREDICTION METHOD, AND PROGRAM**

GENEHMIGUNGSVORHERSAGEVORRICHTUNG, GENEHMIGUNGSVORHERSAGEVERFAHREN UND PROGRAMM

DISPOSITIF DE PRÉDICTION D'APPROBATION, PROCÉDÉ DE PRÉDICTION D'APPROBATION ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.10.2012 JP 2012219730**

(43) Date of publication of application:
**12.08.2015 Bulletin 2015/33**

(73) Proprietor: **Japan Science and Technology Agency**
**Kawaguchi-shi**
**Saitama 332-0012 (JP)**

(72) Inventors:
• **DA SILVA LOPES, Tiago Jose**
**Tokyo 108-8639 (JP)**
• **KITANO, Hiroaki**
**Tokyo 108-8639 (JP)**
• **KAWAOKA, Yoshihiro**
**Tokyo 108-8639 (JP)**

(74) Representative: **Plasseraud IP**
**66, rue de la Chaussée d'Antin**
**75440 Paris Cedex 09 (FR)**

(56) References cited:
WO-A1-03/077159    WO-A1-2005/069188
JP-A- 2006 146 380    US-A1- 2011 196 620

• **MICHAEL J. KEISER ET AL.:** 'Predicting new molecular targets for known drugs' NATURE vol. 462, November 2009, XP055257869
• **MONICA CAMPILLOS ET AL.:** 'Drug Target Identification Using Side-Effect Similarity' SCIENCE vol. 321, July 2008, XP002534673
• **EUGEN LOUNKINE ET AL.:** 'Large-scale prediction and testing of drug activity on side-effect targets' NATURE vol. 486, 21 June 2012, XP055257873
• **JUNJI SUZUKI ET AL.:** 'Knowledge Discovery from Protein-Protein-Interaction Data though Network Analysis' PROCEEDINGS OF THE 21ST ANNUAL CONFERENCE OF JSAI June 2007, pages 1 - 2, XP008179454

**Description**

TECHNICAL FIELD

The present invention relates to an approval prediction apparatus, an approval prediction method, and a computer program product.

BACKGROUND ART

Conventional technologies for predicting off-targets and side effects of existing compounds have been disclosed.

As for the identification of protein functions according to Non Patent Literature 1, a technology for detecting off-targets of drugs by grouping proteins according to the similarities between their ligands has been disclosed where unexpected relations between drugs, such as methadone, emetine and loperamide, are found in that they antagonize receptors not previously reported in the literature.

As for the identification of drug targets according to Non Patent Literature 2, a technology has been disclosed where off-target effects are investigated using the side-effects caused by marketed drugs as a starting point and drugs are grouped according to their side effects to group the drugs having indications and structures, which makes it possible to determine additional protein targets for the drugs that were not known before.

As for the prediction of new molecular targets of known drugs according to Non Patent Literature 3, a technology has been disclosed where proteins are grouped according to the similarity of their ligands and off-target effects are investigated to find other targets in addition to the reported targets.

As for the prediction of drug target interaction networks according to Non Patent Literature 4, a technology has been disclosed where information on protein sequences and drug targets are correlated to newly create a resource referred to as "pharmacological space" and, using this resource, known additional targets for known drugs are revealed and the drug targets are classified into four classes of enzymes, ion channels, G-protein-coupled and nuclear receptors.

As for the large-scale prediction of drug activity according to Non Patent Literature 5, a technology has been disclosed where a drug target-adverse effect network that is used to predict and explain the side effects of marketed drugs is created and, from various unintended interaction between drugs and certain proteins, adverse effects that cannot be explained before can be discovered.

The drug induced liver injury prediction system according to Non Patent Literature 6 is a prediction system for identifying a compound with a high potential to cause liver injury, and a technology has been disclosed where a prediction target is limited to liver and a characteristic of a given type of compound to be likely to cause liver injury is predicted based on the investigations according to scientific literatures. The drug induced liver injury prediction system predicts some proteins and pathways having a potential to cause harmful effects to liver.

CITATION LIST

NON PATENT LITERATURE

Non Patent Literature 1: Keiser MJ, Roth BL, Armbruster BN, Ernsberger P, Irwin JJ, Shoichet BK. (2007) Relating protein pharmacology by ligand chemistry, Nature Biotechnology, 25, 197-206.
Non Patent Literature 2: Campillos M, Kuhn M, Gavin AC, Jensen LJ, Bork P. (2008) Drug Target Identification Using Side-Effect Similarity, Science, 321, 263-266.
Non Patent Literature 3: Keiser MJ, Setola V, Irwin JJ, Laggner C, Abbas AI, Hufeisen SJ, Jensen NH, Kuijer MB, Matos RC, Tran TB, Whaley R, Glennon RA, Hert J, Thomas KL, Edwards DD, Shoichet BK, Roth BL. (2009) Predicting new molecular targets for known drugs, Nature, 462, 175-181.
Non Patent Literature 4: Yamanishi Y, Araki M, Gutteridge A, Honda W, Kanehisa M (2008) Prediction of drug target interaction networks from the integration of chemical and genomic spaces, Bioinformatics, 24, i232-i240.
Non Patent Literature 5: Lounkine E, Keiser MJ, Whitebread S, Mikhailov D, Hamon J, Jenkins JL, Lavan P, Weber E, Doak AK, Cote S, Shoichet BK, Urban L. (2012) Large-scale prediction and testing of drug activity on side-effect targets, Nature, 486, 361-367.
Non Patent Literature 6: Liu Z, Shi Q, Ding D, Kelly R, Fang H, et al. (2011) Translating Clinical Findings into Knowledge in Drug Safety Evaluation - Drug Induced Liver Injury Prediction System (DILIps). PLoS Comput Biol 7(12): e1002310.

SUMMARY OF INVENTION

PROBLEM TO BE SOLVED BY THE INVENTION

The conventional drug target prediction technologies described in Non Patent Literature 1 to 6, however, have a problem in that they do not make it possible to quantify the probability of drug approval based on the properties of target proteins.

The present invention was made in view of the above-described problem, and an object of the present invention is to provide an approval prediction apparatus, an approval prediction method, and a computer program product that allow to quantify the probability of drug approval or rejection upon evaluation.

MEANS FOR SOLVING PROBLEM

In order to attain this object, an approval prediction apparatus according to one aspect of the present invention is proposed as set forth in the appended claims.

Approval prediction methods according to other aspects of the present invention are also proposed as set forth in the appended claims.

Computer program products according to still other aspects of the present invention are proposed as set forth in the appended claims.

EFFECT OF THE INVENTION

According to one aspect of the present invention, similarity centrality measures that are centrality measures containing the degree centrality, betweenness centrality, closeness centrality, and Burt's constraint of proteins that a protein similarity network includes are calculated, interaction centrality measures that are centrality measures containing the degree centrality, betweenness centrality, closeness centrality, and Burt's constraint of the proteins that the protein-protein interaction network includes are calculated, a rejection score that represents probability of a compound to be validated to be classified as a rejected drug is calculated using classifiers that use, as training data, the approval attributes of the respective drugs, the sum and average of the calculated similarity centrality measures per target for each drug, and the sum and average of the calculated interaction centrality measures per target for each drug, and the calculated rejection score is output via the output unit. The present invention thus provides an advantage that taking the properties of all proteins into account as targets for a compound allows its applications for prediction of approval or rejection of several target compounds. The present invention further provides an advantage that scoring the probability of candidate compounds to cause undesirable side-effects using machine learning classifiers allows its use at early stages of drug development and helps prioritizing compounds with higher probability of approval.

According to another aspect of the present invention, similarity centrality measures that are centrality measures containing the degree centrality, betweenness centrality, closeness centrality, and Burt's constraint of the proteins that the protein similarity network includes are calculated, based on the approval attributes of the drugs targeting the proteins that the protein similarity network includes, a determination result representing whether the proteins to be validated, which are proteins that the similarity network includes, are within a range of targets of approved drugs or a range of targets of rejected drugs, is obtained using the calculated similarity centrality measures of the proteins to be validated, and the obtained determination result is output via the output unit. Accordingly, the present invention provides an advantage that it is possible to specify the characteristics of individual proteins and determine whether there is probability that harmful effects would be produced. The invention further provides an advantage that it can be used for technologies for siRNA based therapies, evaluating individual targets, such as single-target compounds (aka 'magic bullets') and modulating the activity of single specific proteins.

According to still another aspect of the present invention, the protein similarity network including the proteins between which the similarity is detected is created when the similarity is detected between the proteins using a signature-based algorithm, and the similarity network information on the protein similarity network is stored. Accordingly, the invention provides an advantage that it is possible to provide network data with more considerable similarity than that of the conventional publically-available network data.

According to still another aspect of the present invention, based on the approval attributes of the drugs targeting the proteins that the protein similarity network includes, a determination result representing that the proteins to be validated are within the range of targets of rejected drugs is generated when the degree centrality contained in the calculated similarity centrality measures of the proteins to be validated is high, the closeness centrality is low, and the Burt's constraint is extremely low. Accordingly, the invention provides an advantage that it is possible to accurately identify proteins prone to unspecific binding and side-effects.

BRIEF DESCRIPTION OF DRAWINGS

FIG. 1 is a flowchart of the basic idea of an embodiment.
FIG. 2 is a flowchart of the basic idea of the embodiment.
FIG. 3 is a block diagram of an exemplary configuration of an approval prediction apparatus according to the embodiment.
FIG. 4 is a flowchart of an exemplary processing performed by the approval prediction apparatus according to the embodiment.
FIG. 5 is a diagram of exemplary sequence information according to the embodiment.
FIG. 6 is a diagram of exemplary similarity network information according to the embodiment.
FIG. 7 is a diagram of exemplary Burt's constraint according to the embodiment.
FIG. 8 is a table of exemplary centrality measures of proteins according to the embodiment.
FIG. 9 is a table of exemplary information that is stored in a drug target database according to the embodiment.
FIG. 10 is a diagram of exemplary centrality measures of an approved and rejected drug target according to the embodiment.
FIG. 11 is a table of exemplary interaction network information according to the embodiment.
FIG. 12 is a graph of exemplary improvement of the performance of classifiers according to the embodiment.
FIG. 13 is a graph of exemplary accuracy of classification by classifiers according to the embodiment.
FIG. 14 is a table of exemplary classifiers according to the embodiment.
FIG. 15 is a table of exemplary output information according to the embodiment.

MODE(S) FOR CARRYING OUT THE INVENTION

An embodiment of an approval prediction apparatus, an approval prediction method, and a computer program product according to the present invention will be explained in detail below according to the drawings. The embodiment does not limit the invention.

Overview of Embodiment of Invention

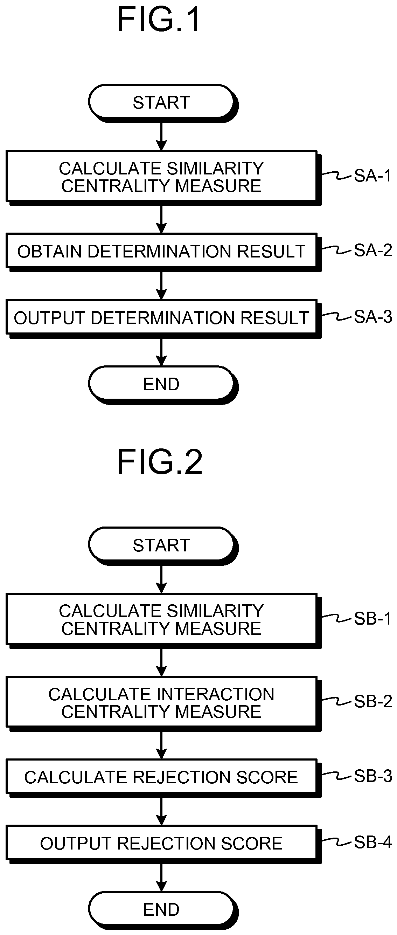

An overview of the embodiment of the invention will be explained with reference to FIGS. 1 and 2 and then a configuration and processing according to the embodiment will be explained in detail below.

Overview (1)

With reference to FIG. 1, an exemplary overview of the embodiment of the invention will be explained. FIG. 1 is a flowchart of the basic idea of the embodiment. Schematically, the embodiment has the following basic features.
Specifically, as shown in FIG. 1, the control unit of the approval prediction apparatus according to the embodiment calculates similarity centrality measures that are centrality measures containing the degree centrality, betweenness centrality, closeness centrality, and Burt's constraint of proteins that a protein similarity network includes (step SA-1).
Based on the approval attributes of drugs targeting proteins that the protein similarity network includes, a control unit of the approval prediction apparatus obtains a determination result representing whether the proteins to be validated, which are proteins that the similarity network includes, are within the range of targets of approved drugs or the range of targets of rejected drugs, using the similarity centrality measures of the proteins to be validated that are calculated at step SA-1 (step SA-2).
The control unit of the approval prediction apparatus outputs the determination result obtained at step SA-2 (step SA-3) via an output unit and ends the processing.
This is the explanation of Overview (1).

Overview (2)

With reference to FIG. 2, an exemplary overview of the embodiment of the invention will be explained. FIG. 2 is a flowchart of the basic idea of the embodiment.
As shown in FIG. 2, the control unit of the approval prediction apparatus according to the embodiment calculates similarity centrality measures that are centrality measures containing the degree centrality, betweenness centrality, closeness centrality, and Burt's constraint of proteins that a protein similarity network includes (step SB-1).
The control unit of the approval prediction apparatus according to the embodiment then calculates interaction centrality measures that are centrality measures containing the degree centrality, betweenness centrality, closeness centrality, and Burt's constraint of proteins that a protein-protein interaction network includes (step SB-2).

The control unit of the approval prediction apparatus calculates a rejection score that represents probability that a compound to be validated is classified as a rejected drug, using classifiers that use, as training data, the approval attribute of each drug, the sum and average of the similarity centrality measures per target for each drug that are calculated at step SB-1, and the sum and average of the interaction centrality measures per target for each drug that are calculated at step SB-2 (step SB-3).

The control unit of the approval prediction apparatus outputs the rejection score that is calculated at step SB-3 (step SB-4) via the output unit and ends the processing.

This is the explanation of the overview of the embodiment.

Configuration of Approval Prediction Apparatus 100

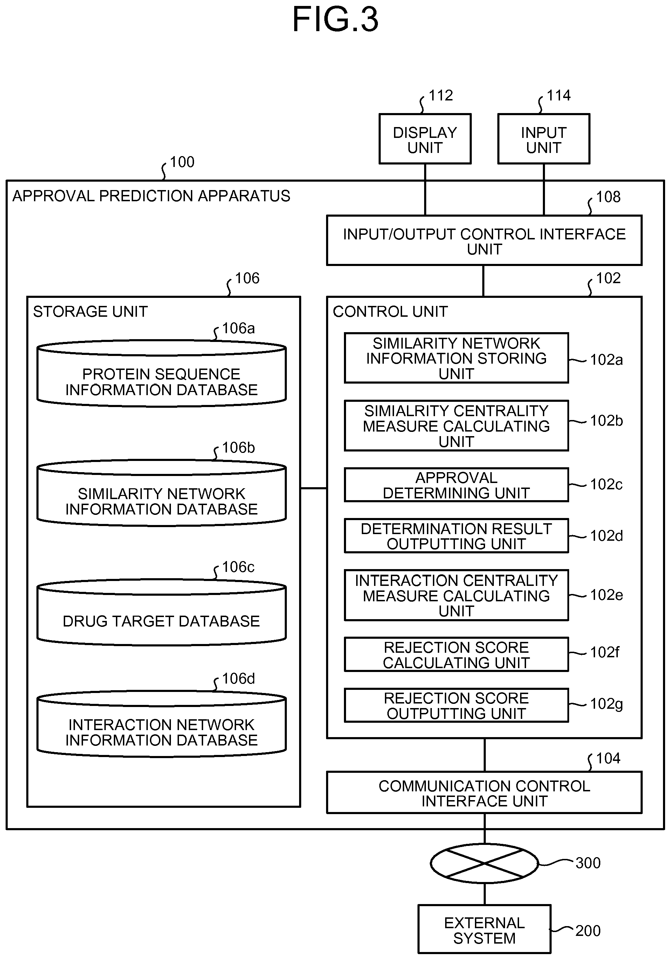

Details of the configuration of the approval prediction apparatus 100 according to the embodiment will be explained below with reference to FIG. 3. FIG. 3 is a block diagram of an exemplary configuration of the approval prediction apparatus 100 according to the embodiment and schematically illustrates only components relevant to the invention. In the approval prediction apparatus 100 according to the embodiment, all components are provided in a single enclosure, and one that independently performs processing (stand-alone) will be explained as the approval prediction apparatus 100; however, in addition to this example, it may be one (e.g., cloud computing) in which components are provided respectively in independent enclosures and are connected via a network 300, or the like, to configure an apparatus as a single concept.

In FIG. 3, an external system 200 and the approval prediction apparatus 100 are interconnected via the network 300. The external system 200 may have a function of providing any one or both of an external database relating to any one, some, or all of protein sequence information, drug information, drug target information, and protein-protein interaction information, and a website for implementing a user interface, or the like.

The external system 200 may be configured as a web server or as an ASP server. The hardware configuration of the external system 200 may include an information processing device, such as a marketed work station or a personal computer, and its peripheral devices. Each function of the external system 200 may be implemented by a CPU, a disk device, a memory device, an input device, an output device, and a communication control device of the hardware configuration of the external system 200 and a computer program for controlling them.

The network 300 has a function of interconnecting the approval prediction apparatus 100 and the external system 200. The network 300 is, for example, the Internet.

The approval prediction apparatus 100 schematically includes a control unit 102, a communication control interface unit 104, a storage unit 106, and an input/output control interface unit 108. The approval prediction apparatus 100 may further include an output unit, including the display unit 112, and an input unit 114. The output unit may further include an audio output unit and a print output unit. The control unit 102 is a CPU, or the like, that generally controls the whole approval prediction apparatus 100. The communication control interface unit 104 is an interface that is connected to a communication device (not shown), such as a router, connected to a communication line, or the like, and the input/output control interface unit 108 is an interface that is connected to the output unit and the input unit 114. The storage unit 106 is a device that stores various databases and tables. The units of the approval prediction apparatus 100 are communicably connected to one another via arbitrary communication paths. Furthermore, the approval prediction apparatus 100 is communicably connected to the network 300 via a communication device, such as a router, or a wired or wireless communication line, such as a dedicated line.

The various databases and tables stored in the storage unit 106 (a protein sequence information database 106a, a similarity network information database 106b, a drug target database 106c, and an interaction network information database 106d) are storage units, such as a fixed disk device. For example, the storage unit 106 stores various programs used for various types of processing, tables, files, databases, and webpages.

From among the components of the storage unit 106, the protein sequence information database 106a is a protein sequence information storage unit that stores sequence information on protein amino acid sequences. The amino acid sequences may be human protein amino acid sequences. The sequence information may be in FASTA format. The sequence information is previously stored in the protein sequence information database 106a. The control unit 102 of the approval prediction apparatus 100 may, periodically and/or according to the processing performed by the control unit 102, download the latest data via the network 300 from the external system 200 (e.g., an NCBI or an UNIPROT) and update the sequence information stored in the protein sequence information database 106a.

The similarity network information database 106b is a similarity network information storage unit that stores protein similarity network information on a protein similarity network (PSIN) including proteins having similarity.

The drug target database 106c is a drug target storage unit that stores drug information including approval attributes of drugs on approval or rejection and protein quality information on proteins targeted by the drugs in association with each other. The rejected drugs may be drugs that are, according to the embodiment, withdrawn or illicit drugs in drug approval that are regarded as a group of problematic drugs. In other words, problematic drugs may be drugs that have to be withdrawn from the markets because of their harmful effects or illegal drugs (e.g., stimulants or hallucinogens) that are socially prohibited and that have to be distinguished from approved drugs. The drug information and the protein information on drug approval are stored beforehand in the drug target database 106c and the control unit 102 of the approval prediction apparatus 100 periodically, and/or according to the processing performed by the control unit 102, downloads the latest data from the external system 200 (e.g., Drugbank (http://www.drugbank.ca/)) via the network 300 and updates the drug information and the protein information on drug approval that are stored in the drug target database 106c.

The interaction network information database 106d is an interaction network information storage unit that stores interaction network information on a protein-protein interaction network (PPI) constructed according to the interactions between proteins. The interaction network information is stored beforehand in the interaction network information database 106d, and the control unit 102 of the approval prediction apparatus 100 periodically, and/or according to the processing performed by the control unit 102, downloads the latest data from the external system 200 (e.g., HIPPIE (http://cbdm.mdc-berlin.de/tools/hippie/)) via the network 300 and updates the interaction network information that is stored in the interaction network information database 106d.

The communication control interface unit 104 performs communication control between the approval prediction apparatus 100 and the network 300 (or a communication device such as a router). In other words, the communication control interface unit 104 has a function of communicating data with the external system 200 and other terminals via communication lines.

The input/output control interface unit 108 controls the output unit (display unit 112) and the input unit 114.

The display unit 112 may be a display unit (such as a display, monitor, or a touch panel configured of liquid crystals or organic EL) that displays a display screen of an application or the like. The input unit 114 may be, for example, a key input unit, a touch panel, a control pad (e.g., a touch pad or gamepad), a mouse, a keyboard, or a microphone. It may be, as an audio output unit, for example, a speaker. It may be, as a print output unit, for example, a printer.

The control unit 102 in FIG. 3 has an internal memory for storing control programs of an operating system (OS), etc., programs that define various process procedures, and necessary data. The control unit 102 performs information processing for performing various processes according to the programs, etc. The control unit 102 includes, as functional concepts, a similarity network information storing unit 102a, a similarity centrality measure calculating unit 102b, an approval determining unit 102c, a determination result outputting unit 102d, an interaction centrality measure calculating unit 102e, a rejection score calculating unit 102f, and a rejection score outputting unit 102g.

The similarity network information storing unit 102a is a similarity network information storing unit that, when similarity is detected between proteins using a signature-based algorithm and based on the sequence information stored in the protein sequence information database 106a, creates a protein similarity network (PSIN) including the proteins between which the similarity is detected and stores the similarity network information on the protein similarity network in the similarity network information database 106b.

The similarity centrality measure calculating unit 102b is a similarity centrality measure calculating unit that calculates, based on the similarity network information stored in the similarity network information database 106b, similarity centrality measures that are centrality measures containing the degree centrality, betweenness centrality, closeness centrality, and Burt's constraint of the proteins that the protein similarity network includes. The degree centrality is an index representing how much the node is directly connected to other nodes (how many direct connections to other nodes the node has) in the network. The betweenness centrality measures the centrality of the protein network by counting the number of shortest paths that have to be passed to connect to other nodes in the network. The closeness centrality measures how many steps are necessary to reach every other node in the network. The Burt's constraint is an index proposed in a sociological context to study the positions and advantages of individuals within a group.

The approval determining unit 102c is an approval determining unit that, based on the approval attributes of the drugs targeting the proteins according to the protein information stored in the drug target database 106c, which are the proteins that the protein similarity network includes, obtains a determination result representing whether the proteins to be validated that the protein similarity network includes are within the range of targets of approved drugs or the range of targets of rejected drugs, using the centrality measures of the proteins to be validated that are calculated by the similarity centrality measure calculating unit 102b. The approval determining unit 102c may, based on the approval attributes of the drugs targeting the proteins according to the protein information stored in the drug target database 106c, which are the proteins that the protein similarity network includes, generate a determination result representing that the proteins to be validated are within the range of targets of rejected drugs when the degree centrality contained in the similarity centrality measures of the proteins to be validated that are calculated by the similarity centrality measure calculating unit 102b is high, the closeness centrality is low, and the Burt's constraint is extremely low. The proteins to be validated may be according to the protein information that is input by the user via the input unit 114.

The determination result outputting unit 102d is a determination result outputting unit that outputs the determination result obtained by the approval determining unit 102c via the output unit. The determination result outputting unit 102d may display the determination result on the display unit 112. The determination result outputting unit 102d may output the determination result via a print output unit.

The interaction centrality measure calculating unit 102e is an interaction centrality measure calculating unit that calculates, based on the interaction network information that is stored in the interaction network information database 106d, interaction centrality measures that are centrality measures containing the degree centrality, betweenness centrality, closeness centrality, and Burt's constraint of the proteins that the protein-protein interaction network includes.

The rejection score calculating unit 102f is a rejection score calculating unit that calculates a rejection score that represents probability that a compound to be validated is classified as a rejected drug, using classifiers that use, as training data, the approval attribute of each drug stored in the drug target database 106c, the sum and average of the similarity centrality measures per target for each drug that are calculated by the similarity centrality measure calculating unit 102b, and the sum and average of the interaction centrality measures per target for each drug that are calculated by the interaction centrality measure calculating unit 102e. The compound (drug) to be validated may be based on the compound information that is input by the user via the input unit 114.

The rejection score outputting unit 102g is a rejection score outputting unit that outputs the rejection score, which is calculated by the rejection score calculating unit 102f, via the output unit. The rejection score outputting unit 102g may display the rejection score on the display unit 112. The rejection score outputting unit 102g may output the rejection score via a print output unit.

The explanation of the exemplary configuration of the approval prediction apparatus 100 according to the embodiment configured as descried above ends here.

Processing Performed by Approval Prediction Apparatus 100

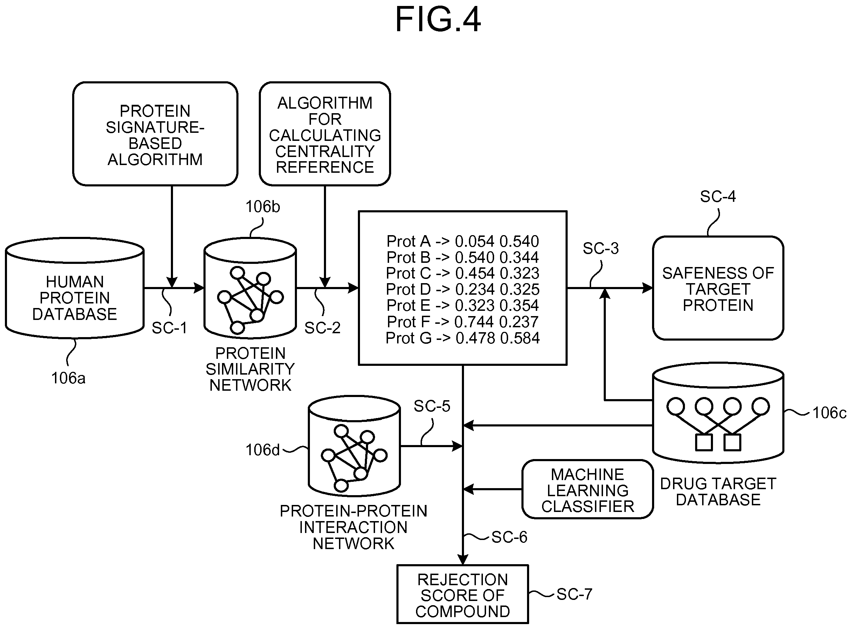

Details of the processing performed by the approval prediction apparatus 100 according to the embodiment configured as described above will be explained below with reference to FIGS. 4 to 15. FIG. 4 is a flowchart of exemplary processing performed by the approval prediction apparatus 100 according to the embodiment.

As shown in FIG. 4, when similarity is detected between proteins with a protein signature-based algorithm for finding similarity between protein homologs and based on the sequence information stored in the human protein database (protein sequence information database) 106a, the similarity network information storing unit 102a creates a protein similarity network (PSIN) including the proteins between which the similarity is detected and stores the similarity network information on the protein similarity network in the similarity network information database 106b (step SC-1). When the PSI-BLAST tool (Schaffer, et al., 2001) to query and compare each of the 22,000 human proteins to the NCBI human protein database is used in order to find similar proteins, distinct from previous studies (Atkinson, et al., 2009; Camoglu, et al., 2006; Rattei, et al., 2010; Valavanis, et al., 2010; Weston, et al., 2004; Zhang and Grigorov, 2006), the results representing that interaction (meaning that when protein A is queried and protein B is identified to be similar, protein B is queried and protein A is identified to be similar) are obtained. According to this result, the similarity network information storing unit 102a creates a new protein similarity network (PSIN) using graph theory representation. In the protein similarity network (PSIN), the nodes represent proteins and two nodes are connected by an edge only if the nodes share considerable protein sequence similarity and also, bidirectional hits (i.e., protein A is identified to be similar to protein B and vice-versa) are verified. Accordingly, the similarity network information storing unit 102a creates a protein similarity network (PSIN) containing 19,721 nodes and 776,598 edges.

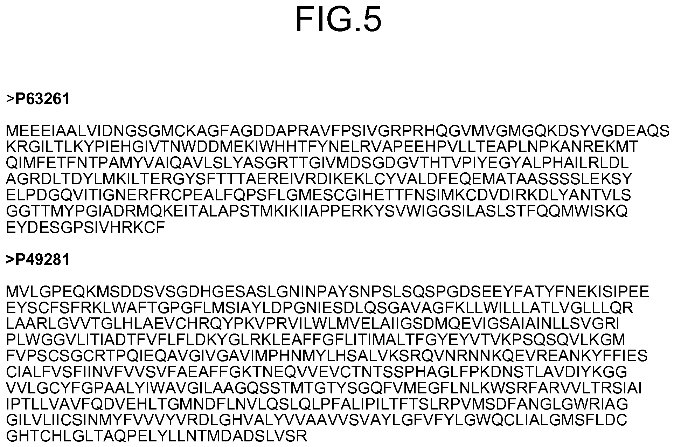

With reference to FIG. 5, exemplary sequence information according to the embodiment will be explained here. FIG. 5 is a diagram of the exemplary sequence information according to the embodiment.

As shown in FIG. 5, the sequence information stored in the protein sequence information database 106a may be protein sequence information on human proteins in FASTA format, such as P63261 and P49281.

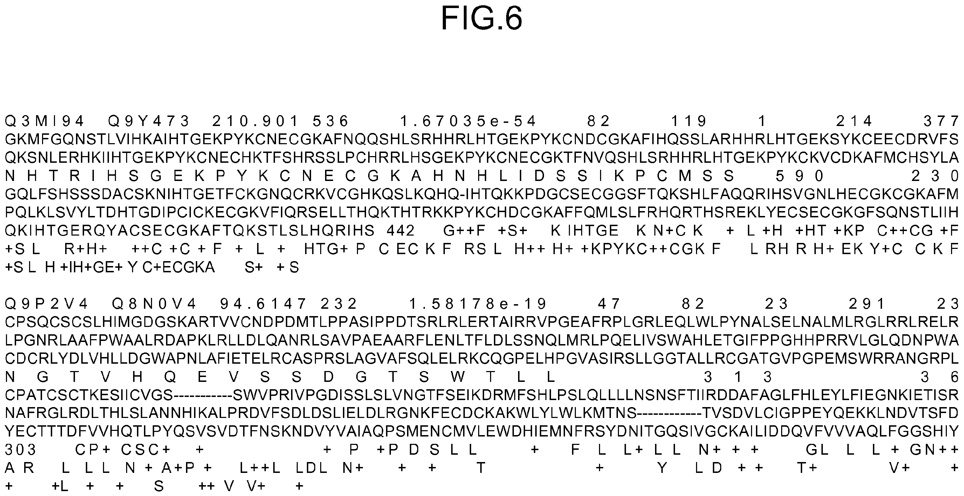

With reference to FIG. 6, the exemplary similarity network information according to the embodiment will be explained. FIG. 6 is a diagram of the exemplary similarity network information according to the embodiment.

As shown in FIG. 6, the similarity network information according to the embodiment may contain the names of proteins, the names of proteins similar to the protein (neighbours), the sequence scores, and the sequence information on the region where two proteins are similar. FIG. 6 exemplarily shows the similarity network information on the similarity between Q3MI94 and Q9Y473 and the similarity network information on Q9P2V4 and Q8N0V4.

The following refers back to FIG. 4. Based on the similarity network information stored in the similarity network information database 106b and by using the algorithm for calculating a centrality reference, the similarity centrality measure calculating unit 102b calculates the degree centrality, betweenness centrality, closeness centrality and Burt's constraint of proteins that the protein similarity network (PSIN) includes (step SC-2).

The centrality measures of the proteins that the PSIN includes according to the embodiment will be explained here. The similarity centrality measure calculating unit 102b calculates the degree centrality that is an index representing how much the node is directly connected to nodes in the PSIN, ranging from 1 (the least connected) to 441 (the most connected) in the PSIN.

The similarity centrality measure calculating unit 102b calculates the betweenness centrality B(v) using the following Expression (1) formed of $S_{ij}$ denoting the number of shortest paths between a node i and a node j, and $S_{ij}(v)$ denoting the fraction of shortest paths passing through a node v.

Expression 1

$$B(v) = \sum \frac{S_{ij}(v)}{S_{ij}}, \qquad with\ i \neq j, v \neq i\ and\ v \neq j \qquad \cdots(1)$$

The similarity centrality measure calculating unit 102b calculates the closeness centrality C(v) using the following Expression (2) formed of d(v,i) denoting the distance represented at the step between a node v and the node i.

Expression 2

$$C(v) = \frac{1}{\sum d(v, i)}, \qquad with\ i \neq v \qquad \cdots(2)$$

The similarity centrality measure calculating unit 102b calculates the Burt's constraint C(i) using the following Expression (3) formed of $p_{iq}p_{qj}$ denoting a product of the proportional strength of the node j's relationship with the node i and the proportional strength of the node j's relationship with the node q.

Expression 3

$$C(i) = \sum_{j} (p_{ij} + \sum_{q} p_{iq}p_{qj})^2, \qquad with\ q \neq i, j, and\ j \neq i \qquad \cdots(3)$$

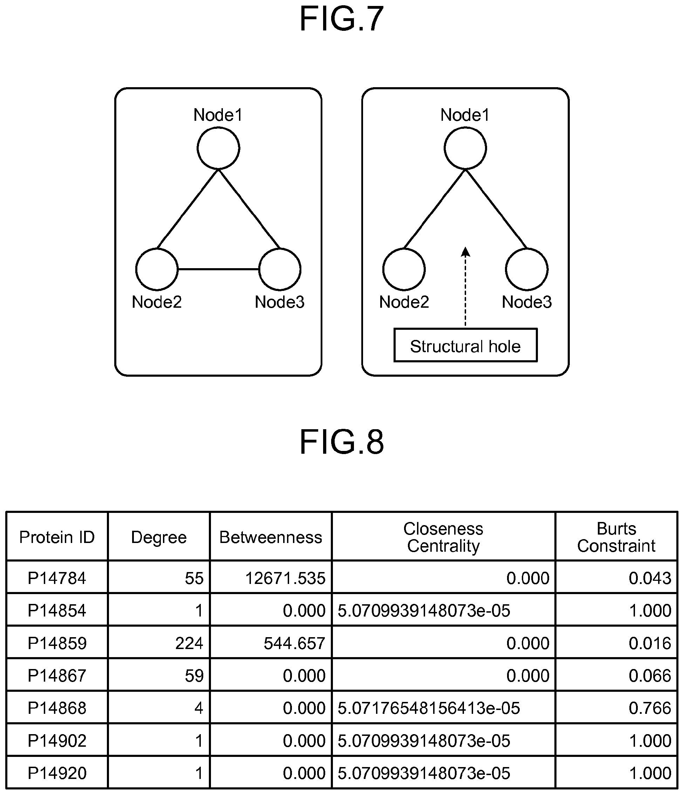

With reference to FIG. 7, the Burt's constraint according to the embodiment will be explained. FIG. 7 is a diagram of the exemplary Burt's constraint according to the embodiment.

The Burt's constraint is a method proposed in a sociological context to study the positions and advantages of individuals within a group. If the nodes are individuals in FIG. 7, all nodes have alternative connections and thus are able to negotiate or bargain with others according to the left diagram in FIG. 7. On the other hand, if there is a structural hole as shown in the right diagram in FIG. 7, Node 1 is in a better position for negotiation, because Node 2 and Node 3 are not able to be aware of each other's presence. The embodiment applies it to a similar context of nodes that are proteins so that proteins (nodes) with small Burt's constraint are generally those with several domains, located between different protein families, and proteins (nodes) with large Burt's constraint represent a few neighbors and sequence similarity.

With reference to FIG. 8, exemplary centrality measures of proteins according to the embodiment will be explained. FIG. 8 is a table of the exemplary centrality measures of proteins according to the embodiment.

As shown in FIG. 8, the similarity centrality measure calculating unit 102b may calculate, as centrality measures, the degree centrality, betweenness centrality, closeness centrality, and Burt's constraint of proteins (P14784, P14854, P14859, P14867, P14868, P14902, and P14920) that the PSIN includes and output a list of the centrality measures.

The following refers back to FIG. 4. Based on the approval attributes of drugs targeting the proteins according to the protein information stored in the drug target database 106c, which are proteins that the protein similarity network includes, the approval determining unit 102c obtains a determination result representing whether proteins to be validated that the protein similarity network includes are within the range of targets of approved drugs or the range of targets of rejected drugs (safeness of targeted proteins), using the degree centrality, betweenness centrality, closeness centrality, and Burt's constraint of the proteins to be validated that are calculated by the similarity centrality measure calculating unit 102b at step SC-2 (step SC-3). In other words, the approval determining unit 102c may require the centrality measures of the proteins that the protein similarity network includes and the list stored in the drug target database 106c and determine the ranges of values assuming targets of approved drugs and targets of rejected (withdrawn and illicit) drugs. At this step, only individual proteins, not the complete set of proteins that can be targeted by a compound, are considered. The motivation to determine the characteristics of individual drug targets is that single-target compounds (magic bullets) and siRNA based therapies are designed to inhibit only one target, and hence, it is essential to select the targets on the assumption that therapeutic inhibition of targets is safe.

The approval determining unit 102c may, based on the approval attributes of the drugs targeting the proteins according to the protein information stored in the drug target database 106c, which are proteins that the protein similarity network includes, generate a determination result representing that the proteins to be validated are within the range of targets of rejected drugs when the degree centrality contained in the similarity centrality measures of the proteins to be validated, which are the similarity centrality measures calculated by the similarity centrality measure calculating unit 102b at step SC-2, is high, the closeness centrality is low, and the Burt's constraint is extremely low.

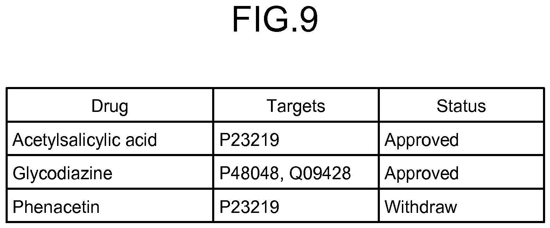

With reference to FIG. 9, exemplary information stored in the drug target database 106c according to the embodiment will be described. FIG. 9 is a diagram of the exemplary information stored in the drug target database 106c according to the embodiment.

As shown in FIG. 9, the information stored in the drug target database 106c according to the embodiment may contain the names of drugs (drug), names of proteins targeted by the drugs (targets), and approval attributes (status) on approval or rejection of the drugs (by the Japanese Ministry of Health, Labour and Welfare, the US FDA, or the like).

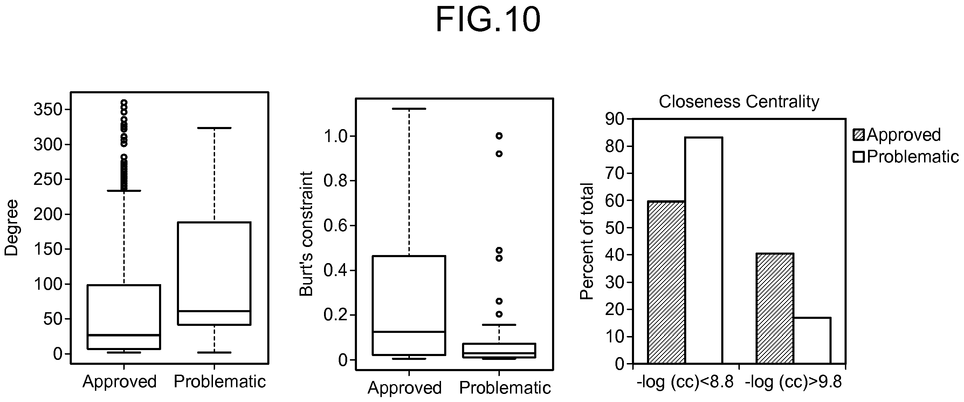

With reference to FIG. 10, exemplary centrality measures of approved or rejected targets according to the embodiment will be explained. FIG. 10 is a diagram of centrality measures of targets of approved and rejected drugs according to the embodiment.

As shown in FIG. 10, proteins targeted by rejected (problematic) drugs may show high degree centrality, significantly lower Burt's constraint, and lower closeness centrality in negative log scale. As shown in FIG. 10, while the targets of approved drugs have structures less shared among many other proteins (low-degree), targets of rejected drugs have structures much shared among several proteins, hence, having features of being prone to unspecific binding and side-effects.

The following refers back to FIG. 4. The determination result outputting unit 102d displays the safeness of the targeted proteins, which is obtained by the approval determining unit 102c, on the display unit 112 (step SC-4). The determination result outputting unit 102d may output the determination result via a print output unit. The determination result outputting unit 102d may output a list that users can query to verify whether the proteins of the user's interest are within the range of safe drug targets or the range of unsafe drug targets.

On the other hand, based on the interaction network information stored in the interaction network information database 106d, the interaction centrality measure calculating unit 102e calculates the degree centrality, betweenness centrality, closeness centrality, and Burt's constraint of the proteins that the protein-protein interaction network (PPI) includes (step SC-5).

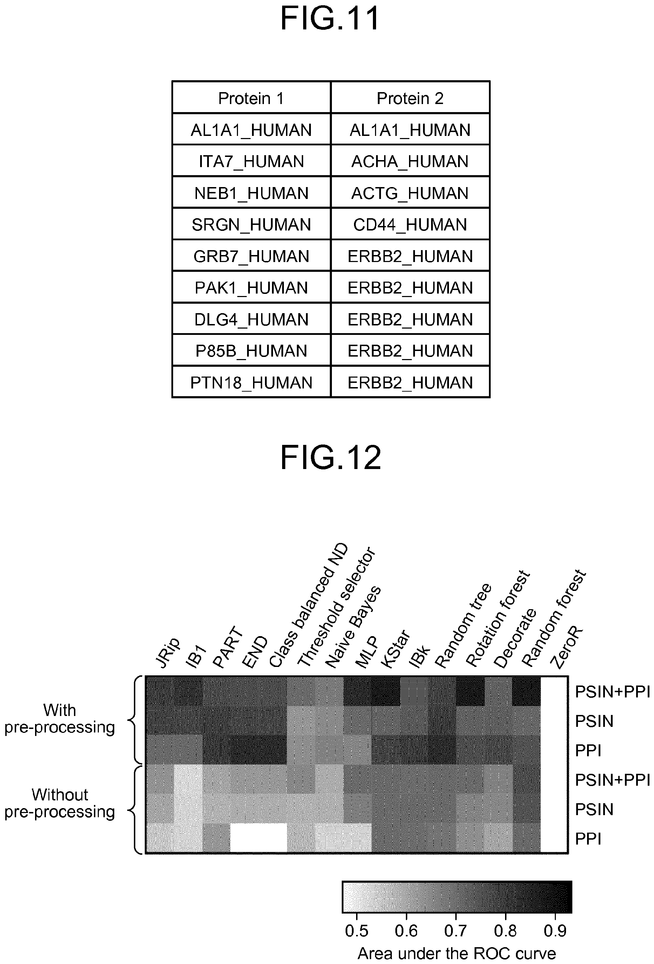

With reference to FIG. 11, exemplary interaction network information according to the embodiment will be explained. FIG. 11 is a diagram of the exemplary interaction network information according to the embodiment.

As shown in FIG. 11, the interaction network information according to the embodiment may contain a list of sets of proteins that physically interact with each other.

The following refers back to FIG. 4. The rejection score calculating unit 102f calculates a rejection score that represents probability that a compound to be validated is classified as a rejected drug, using machine learning classifiers that use, as training data, the approval attributes of each drug stored in the drug target database 106c, the sum and average of the degree centrality, betweenness centrality, closeness centrality, and Burt's constraint per target for each drug that are calculated by the similarity centrality measure calculating unit 102b at step SC-2, and the sum and average of the degree centrality, betweenness centrality, closeness centrality, and Burt's constraint per target for each drug that are calculated by the interaction centrality measure calculating unit 102e at step SC-5(step SC-6). The drug target database 106c reports that most existing drugs (compounds) bind and inhibit the activity of several proteins at once, i.e., reports several drug targets, and hence, it is necessary to consider the centrality measures of all proteins targeted by each compound. The rejection score calculating unit 102f thus calculates, using the protein similarity network (PSIN) and protein-protein interaction network (PPI), the sum and average of the degree centrality, betweenness centrality, closeness centrality, and Burt's constraint per target for each drug and uses eight attributes from the PSIN, eight attributes from the PPI, and one attribute indicating the class of the (approved or rejected) compound as a final data set to be input to the classifiers. The machine learning classifiers may be a set of machine learning classifiers, such as an existing package (Wishart, 2006) like WEKA.

According to the embodiment, using the machine learning classification and classification of (approved and rejected) drugs as a guide for the training and prediction steps, 10-fold cross validation is used to process the final data set. Additionally, according to the embodiment, this step is performed using several different classification algorithms and it is verified that the prediction performance is enhanced in two cases: when pre-processing techniques are used and when centrality measures from the protein similarity network (PSIN) and centrality measures from the protein-protein interaction network (PPI) are used for the same dataset.

The pre-processing according to the embodiment may be performed in the following three steps. It is necessary to first fill the missing values with a unit and mode of the other instances of the synthesized dataset, second increase the number of instances in the smaller class, and finally sample the dataset. It is necessary to collect more samples from samples for much smaller classes because the dataset according to the embodiment is composed of several instances of the approved class and only ~300 examples of the rejected (problematic) class. For this reason, in consideration for the development costs of a new compound, the inconvenience caused by misclassifying an approved drug as a problematic one is smaller than classifying a problematic drug as an approved one. Hence, according to the embodiment, the SMOTE algorithm may be used for over-sampling the smaller class and under-sampling the larger class. This strategy improves the performance of classifiers in datasets with varying sizes. To perform the second step that is resampling, instances may be randomly selected from the dataset, i.e., the same instance could be selected twice. Furthermore, the new dataset may have the same number of instances and attributes as that of the original dataset and there may be 50-60 unique instances.

With reference to FIG. 12, exemplary improvement of the performance of classifiers according to the embodiment will be explained. FIG. 12 is a graph of the exemplary improvement of the performance of classifiers according to the embodiment.

As shown in FIG. 12, regarding the classifiers according to the embodiment, it is possible to considerably improve the sensitivity of the classifiers to the class of problematic drugs by using the pre-processing techniques and using the centrality measures from the PSIN and the centrality measures from the PPI for the same data set.

Furthermore, according to the embodiment, a comparison is made for the prediction power between 15 machine learning classifiers using three different strategies. In a first method, a comparison is made using 10-fold cross validation. In a second method, a comparison is made, dividing the original dataset into a training set and a test set with 70% and 35% of instances, respectively. In the embodiment, drugs are randomly selected for 500 times to make an adjustment to diminish unevenness. When dividing the dataset into a training set and a test set, only the training set is pre-processed.

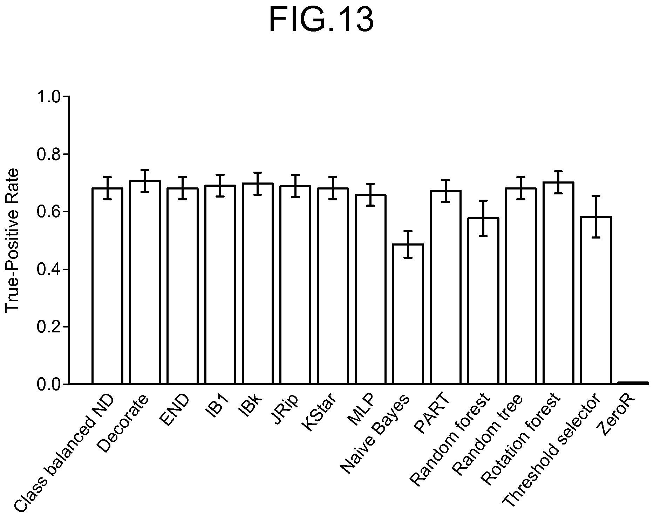

With reference to FIG. 13, exemplary accuracy of classification performed by classifiers according to the embodiment will be explained. FIG. 13 is a graph of the exemplary accuracy of classification performed by classifiers according to the embodiment.

As shown in FIG. 13, for practically measuring the accuracy of the classifiers according to the embodiment, a harmonic mean of the true positive rates for the approved class or problematic class for drugs is used. As shown in FIG. 13, because most classifiers have the same performance (because of optimization of parameters and use of the pre-processing technique), in the embodiment, seven algorithms (such as KSTAR, IBK, Decorate, END ClassBalancedND, JRip and RotationForest) constructed using different principles and implementing best performances are used in order for further safeness prediction of drugs and for the purpose of correcting the biases that all algorithms necessarily have.

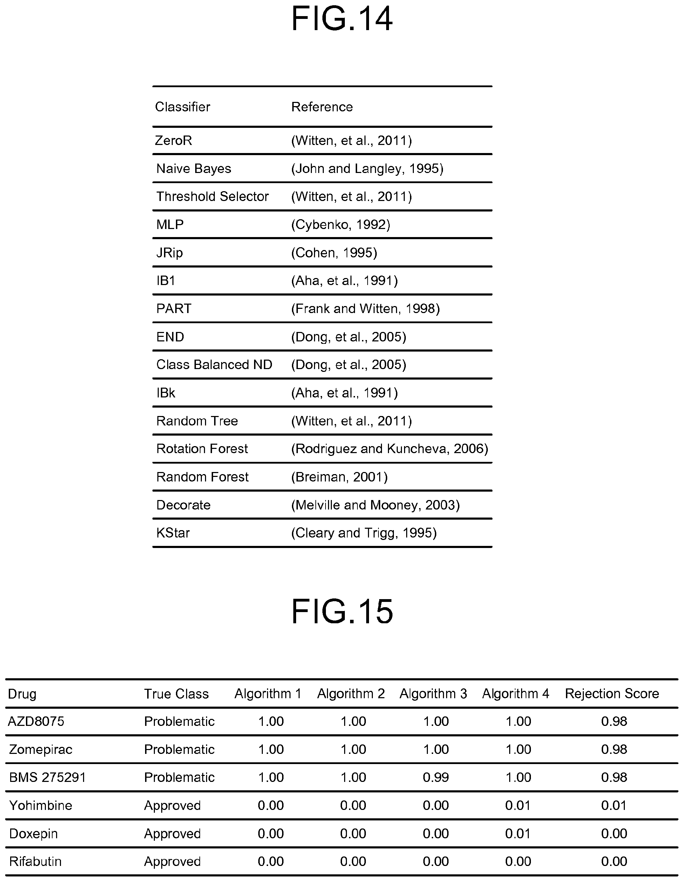

With reference to FIG. 14, exemplary classifiers according to the embodiment will be explained. FIG. 14 is a table of the exemplary classifiers according to the embodiment.

As shown in FIG. 14, because it is verified that KStar, Decorate, Rotation Forest and Random Forest have best performances regardless of whether the original data set is adjusted, these best four algorithms are used for further analysis in the embodiment. During the test phase, when the classifiers categorize instances that have not been detected, these seven optimum algorithms calculate probabilities of each drug belonging to the problematic class and, using the calculated probabilities, create an index, named "rejection score" (RS). According to the embodiment, the value obtained by averaging these probabilities using the contra harmonic mean may be an RS. The value of RS may indicate whether a compound is predicted to be safe (RS close to 0.0) or harmful (RS close to 1.0).

The following refers back to FIG. 4. The rejection score outputting unit 102g displays the rejection score of the compound calculated by the rejection score calculating unit 102f on the display unit 112 (step SC-7) and ends the processing. The rejection score outputting unit 102g may output the rejection score via a print output unit.

With reference to FIG. 15, exemplary output information according to the embodiment will be explained. FIG. 15 is a diagram of the exemplary output information according to the embodiment.

As shown in FIG. 15, the rejection score outputting unit 102g may output a list of drugs and their respective rejection scores (values between 0.00 and 1.00). While problematic drugs have score values close to 1.00, approved drugs have scores close to 0.00. FIG. 15 depicts examples obtained by inputting existing drugs obtained from the Drugbank database. By inputting compounds of interest that could be drug candidates, users can check the rejection score of the standard protein and the compounds. With the method according to the embodiment, the effectiveness of the proposed methodology is verified by accurately distinguishing between existing 1000 approved and rejected drugs.

The explanation of the exemplary processing performed by the approval prediction apparatus 100 according to the embodiment ends here.

Other Embodiments

The embodiment of the present invention is explained above. However, the present invention may be implemented in various different embodiments other than the embodiment described above within a technical scope described in claims.

For example, an example in which the approval prediction apparatus 100 performs the processing as a standalone apparatus is explained. However, the approval prediction apparatus 100 can be configured to perform processes in response to request from a client terminal (having a housing separate from the approval prediction apparatus 100) and return the process results to the client terminal.

All the automatic processes explained in the present embodiment can be, entirely or partially, carried out manually. Similarly, all the manual processes explained in the present embodiment can be, entirely or partially, carried out automatically by a known method.

The process procedures, the control procedures, specific names, information including registration data for each process and various parameters such as search conditions, display example, and database construction, mentioned in the description and drawings can be changed as required unless otherwise specified.

The constituent elements of the approval prediction apparatus 100 are merely conceptual and may not necessarily physically resemble the structures shown in the drawings.

For example, the process functions performed by each device of the approval prediction apparatus 100, especially each of the process functions performed by the control unit 102, can be entirely or partially realized by a CPU and a computer program executed by the CPU or by a hardware using wired logic. The computer program, recorded on a non-transitory tangible computer readable recording medium including programmed commands for causing a computer to execute the method of the present invention, can be mechanically read by the approval prediction apparatus 100 as the situation demands. In other words, the storage unit 106 such as read-only memory (ROM) or hard disk drive (HDD) stores the computer program that can work in coordination with an operating system (OS) to issue commands to the CPU and cause the CPU to perform various processes. The computer program is first loaded to the random access memory (RAM), and forms the control unit in collaboration with the CPU.

Alternatively, the computer program can be stored in any application program server connected to the approval prediction apparatus 100 via the arbitrary network 300, and can be fully or partially loaded as the situation demands.

The computer program may be stored in a computer-readable recording medium, or may be structured as a program product. Here, the "recording medium" includes any "portable physical medium" such as a memory card, a USB (Universal Serial Bus) memory, an SD (Secure Digital) card, a flexible disk, an optical disk, a ROM, an EPROM (Erasable Programmable Read Only Memory), an EEPROM (Electronically Erasable and Programmable Read Only Memory), a CD-ROM (Compact Disk Read Only Memory), an MO (Magneto-Optical disk), a DVD (Digital Versatile Disk), and a Blu-ray Disc.

Computer program refers to a data processing method written in any computer language and written method, and can have software codes and binary codes in any format. The computer program can be in a dispersed form in the form of a plurality of modules or libraries, or can perform various functions in collaboration with a different program such as the OS. Any known configuration in each of the devices according to the embodiment can be used for reading the recording medium. Similarly, any known process procedure for reading or installing the computer program can be used.

Various databases (the protein sequence information database 106a, the similarity network information database 106b, the drug target database 106c, and the interaction network information database 106d) stored in the storage unit 106 are storage units such as a memory device such as a RAM or a ROM, a fixed disk device such as a HDD, a flexible disk, and an optical disk, and stores therein various programs, tables, databases, and web page files used for providing various processing or web sites.

The approval prediction apparatus 100 may be structured as an information processing apparatus such as known personal computers or workstations, or may be structured by connecting any peripheral devices to the information processing apparatus. Furthermore, the approval prediction apparatus 100 may be realized by mounting software (including programs, data, or the like) for causing the information processing apparatus to implement the method according to the invention.

The distribution and integration of the device are not limited to those illustrated in the figures. The device as a whole or in parts can be functionally or physically distributed or integrated in an arbitrary unit according to various attachments or how the device is to be used. That is, any embodiments described above can be combined when implemented, or the embodiments can selectively be implemented.

INDUSTRIAL APPLICABILITY

As described above, according to the present invention, it is possible to provide an approval prediction apparatus, an approval prediction method, and a program that allow to quantify the probability of approval or rejection of drugs, and accordingly it is extremely useful in various fields including medical treatments, pharmaceutics, drug discoveries, and biological researches.

EXPLANATION OF LETTERS OR NUMERALS

100    APPROVAL PREDICTION APPARATUS
102    CONTROL UNIT
102a   SIMILARITY NETWORK INFORMATION STORING UNIT

102b SIMILARITY CENTRALITY MEASURE CALCULATING UNIT
102c APPROVAL DETERMINING UNIT
102d DETERMINATION RESULT OUTPUTTING UNIT
102e INTERACTION CENTRALITY MEASURE CALCULATING UNIT
102f REJECTION SCORE CALCULATING UNIT
102g REJECTION SCORE OUTPUTTING UNIT
104 COMMUNICATION CONTROL INTERFACE UNIT
106 STORAGE UNIT
106a PROTEIN SEQUENCE INFORMATION DATABASE
106b SIMILARITY NETWORK INFORMATION DATABASE
106c DRUG TARGET DATABASE
106d INTERACTION NETWORK INFORMATION DATABASE
108 INPUT/OUTPUT CONTROL INTERFACE UNIT
112 DISPLAY UNIT
114 INPUT UNIT
200 EXTERNAL SYSTEM
300 NETWORK

**Claims**

1. An approval prediction apparatus (100) for drug development comprising an output unit, a storage unit (106), and a control unit (102), wherein
the storage unit (106) includes:
a similarity network information storage unit (106b) configured for storing similarity network information on a protein similarity network that is constructed according to the similarity between proteins, said similarity between proteins being found with a protein signature-based algorithm determining the similarity between two sequence information, said similarity network information being determined as a graph comprising:

- nodes, each node representing a protein, and
- edges, such that two nodes are connected by an edge only if the nodes share considerable protein sequence similarity;

a drug target storage unit (106c) configured for storing drug information containing approval attributes of drugs on approval or rejection and protein information on the proteins targeted by the drugs in association with each other; and an interaction network information storage unit (106d) configured for storing interaction network information on a protein-protein interaction network that is constructed based on interactions between pre-selected proteins, said interaction between proteins meaning that said proteins are similar, said interaction network information being determined as a graph comprising :

- nodes, each node representing a protein, and
- edges such that two nodes are connected if the corresponding proteins have an interaction; and

the control unit (102) includes:
a similarity centrality measure calculating unit (102b) configured for calculating, based on the similarity network information stored in the similarity network information storage unit (106b), similarity centrality measures that are centrality measures containing:

- a degree centrality, defined as an index representing how much the node is directly connected to nodes in the similarity network,
- a betweenness centrality B(v), using the following expression (1) formed of $S_{ij}$ denoting the number of shortest paths between a node i and a node j, and $S_{ij}(v)$ denoting the fraction of shortest paths passing through a node v:

$$(1)\quad \mathrm{B(v)} = \sum \frac{S_{ij}(v)}{S_{ij}} \quad \text{with } i \neq j,\ v \neq i \text{ and } v \neq j,$$

- a closeness centrality C(v), using the following expression (2) formed of d(v,i) denoting the distance represented at the step between a node v and the node i:

$$(2)\quad C(v) = \frac{1}{\sum d(v,i)} \quad \text{with } i \neq v \ ,$$

and

- a Burt's constraint C(i) of the proteins included in the protein similarity network, using the following expression (3) formed of $p_{iq}p_{qj}$ denoting a product of the proportional strength of the node j's relationship with the node i and the proportional strength of the node j's relationship with the node q:

$$(3)\quad C(i) = \sum_j \left(p_{ij} + \sum_q p_{iq}p_{qj}\right)^2 \text{with } q \neq i,j \text{ and } j \neq i;$$

an interaction centrality measure calculating unit (102e) configured for calculating, based on the interaction network information stored in the interaction network information storage unit (106d), interaction centrality measures that are centrality measures containing said degree centrality, betweenness centrality calculated with expression (1), closeness centrality calculated with expression (2), and Burt's constraint of the proteins included in the protein-protein interaction network calculated with expression (3);

a rejection score calculating unit (102f) configured for calculating during the drug development a rejection score that represents a probability of a compound to be validated to be classified as a rejected drug, using classifiers that use, as training data, the approval attributes of the respective drugs stored in the drug target storage unit (106c), the sum and average of the similarity centrality measures per target for each drug that are calculated by the similarity centrality measure calculating unit (102b), and the sum and average of the interaction centrality measures per target for each drug that are calculated by the interaction centrality measure calculating unit (102e), said similarity centrality measures used as parameters for several machine learning classifiers; and

a rejection score outputting unit (102g) configured for outputting, via the output unit, the rejection score that is calculated by the rejection score calculating unit (102f).

2. An approval prediction apparatus (100) for drug development comprising an output unit, a storage unit (106), and a control unit (102), wherein
the storage unit (106) includes:
a similarity network information storage unit (106b) configured for storing similarity network information on a protein similarity network that includes proteins having similarity, said similarity between proteins being found with a protein signature-based algorithm determining the similarity between two sequence information , said similarity network information being determined as a graph comprising:

- nodes, each node representing a protein, and
- edges, such that two nodes are connected by an edge only if the nodes share considerable protein sequence similarity; and

a drug target storage unit (106c) configured for storing drug information containing approval attributes of drugs on approval or rejection and protein information on the proteins targeted by the drugs in association with each other; and
wherein the control unit (102) includes:
a similarity centrality measure calculating unit (102b) configured for calculating, based on the similarity network information stored in the similarity network information storage unit (106b), similarity centrality measures that are centrality measures containing:

- a degree centrality, defined an index representing how much the node is directly connected to nodes in the similarity network, ,
- a betweenness centrality B(v), using the following expression (1) formed of $S_{ij}$ denoting the number of shortest paths between a node i and a node j, and $S_{ij}(v)$ denoting the fraction of shortest paths passing through a node v:

$$(1)\quad B(v) = \sum \frac{S_{ij}(v)}{S_{ij}} \quad \text{with } i \neq j, \ v \neq i \text{ and } v \neq j,$$

- a closeness centrality C(v), using the following expression (2) formed of d(v,i) denoting the distance represented at the step between a node v and the node i:

$$(2) \quad C(v) = \frac{1}{\sum d(v,i)} \quad \text{with } i \neq v \,,$$

and
- a Burt's constraint C(i) of the proteins that the protein similarity network includes, using the following expression (3) formed of $p_{iq}p_{qj}$ denoting a product of the proportional strength of the node j's relationship with the node i and the proportional strength of the node j's relationship with the node q:

$$(3) \quad C(i) = \sum_j \left( p_{ij} + \sum_q p_{iq}p_{qj} \right)^2 \quad \text{with } q \neq i,j \text{ and } j \neq i \;;$$

an approval determining unit (102c) configured for obtaining, based on the approval attributes of the drugs targeting the proteins according to the protein information stored in the drug target storage unit (106c), which are the proteins included in the protein similarity network, during the drug development a determination result representing whether the proteins to be validated, which are proteins that the similarity network includes, are within a range of targets of approved drugs or a range of targets of rejected drugs, using the similarity centrality measures of the proteins to be validated that are calculated by the similarity centrality measure calculating unit (102b); and
a determination result outputting unit (102d) configured for outputting, via the output unit, the determination result that is obtained by the approval determining unit (102c).

3. The approval prediction apparatus (100) according to claim 1 or 2, wherein
the storage unit (106) further includes a protein sequence information storage unit (106a) configured for storing sequence information on amino acid sequences of the proteins, and
the control unit (102) further includes a similarity network information storing unit (102a) configured for creating, when the similarity is detected between the proteins using a signature-based algorithm and based on the sequence information stored in the protein sequence information storage unit (106a), the protein similarity network including the proteins between which the similarity is detected and for storing the similarity network information on the protein similarity network in the similarity network information storage unit (106b).

4. The approval prediction apparatus (100) according to claim 2, wherein, based on the approval attributes of the drugs targeting the proteins according to the protein information stored in the drug target storage unit (106c), which are the proteins that the protein similarity network includes, the approval determining unit (102c) is configured for generating a determination result representing that the proteins to be validated are within the range of targets of rejected drugs when the degree centrality contained in the similarity centrality measures of the proteins to be validated that are calculated by the similarity centrality measure calculating unit (102b) is high, the closeness centrality is low, and the Burt's constraint is extremely low.

5. An approval prediction method for drug development executed by an approval prediction apparatus (100) including an output unit, a storage unit (106), and a control unit (102), wherein
the storage unit (106) includes:
a similarity network information storage unit (106b) configured for storing similarity network information on a protein similarity network that is constructed according to the similarity between proteins, said similarity between proteins being found with a protein signature-based algorithm determining the similarity between two sequence information , said similarity network information being determined as a graph comprising:

- nodes, each node representing a protein, and
- edges, such that two nodes are connected by an edge only if the nodes share considerable protein sequence similarity;

a drug target storage unit (106c) configured for storing drug information containing approval attributes of drugs on approval or rejection and protein information on the proteins targeted by the drugs in association with each other; and
an interaction network information storage unit (106d) configured for storing interaction network information on a protein-protein interaction network that is constructed based on interactions between pre-determined proteins, said interaction between proteins meaning that said proteins are similar , said interaction network information being determined as a graph comprising :

- nodes, each node representing a protein, and
- edges such that two nodes are connected if the corresponding proteins have an interaction;

the method executed by the control unit (102) comprising:

a similarity centrality measure calculating step (SB-1) of, based on the similarity network information stored in the similarity network information storage unit (106b), calculating similarity centrality measures that are centrality measures containing a degree centrality, a betweenness centrality calculated with expression (1), a closeness centrality calculated with expression (2), and a Burt's constraint of the proteins that the protein similarity network includes calculated with expression (3);

an interaction centrality measure calculating step (SB-2) of, based on the interaction network information stored in the interaction network information storage unit (106d), calculating interaction centrality measures that are centrality measures containing:

- the degree centrality, defined as an index representing how much the node is directly connected to nodes in the similarity network,
- betweenness centrality $B(v)$, using the following expression (1) formed of $S_{ij}$ denoting the number of shortest paths between a node i and a node j, and $S_{ij}(v)$ denoting the fraction of shortest paths passing through a node v:

$$(1) \quad B(v) = \sum \frac{S_{ij}(v)}{S_{ij}} \qquad \text{with } i \neq j, \; v \neq i \text{ and } v \neq j,$$

- closeness centrality $C(v)$, using the following expression (2) formed of $d(v,i)$ denoting the distance represented at the step between a node v and the node i:

$$(2) \quad C(v) = \frac{1}{\sum d(v,i)} \qquad \text{with } i \neq v \; ,$$

and
- Burt's constraint $C(i)$ of the proteins that the protein similarity network includes, using the following expression (3) formed of $p_{iq}p_{qj}$ denoting a product of the proportional strength of the node j's relationship with the node i and the proportional strength of the node j's relationship with the node q:

$$(3) \quad C(i) = \sum_j \left( p_{ij} + \sum_q p_{iq}p_{qj} \right)^2 \qquad \text{with } q \neq i, j \text{ and } j \neq i \; ;$$

a rejection score calculating step (SB-3) of calculating during the drug development a rejection score that represents a probability of a compound to be validated to be classified as a rejected drug, using classifiers that use, as training data, the approval attributes of the respective drugs stored in the drug target storage unit (106c), the sum and average of the similarity centrality measures per target for each drug that are calculated at the similarity centrality measure calculating step (SB-1), and the sum and average of the interaction centrality measures per target for each drug that are calculated at the interaction centrality measure calculating step (SB-2), said similarity centrality measures used as parameters for several machine learning classifiers; and

a rejection score outputting step (SB-4) of outputting, via the output unit, the rejection score that is calculated at the rejection score calculating step (SB-3).

6. An approval prediction method for drug development executed by an approval prediction apparatus (100) including an output unit, a storage unit (106), and a control unit (102), wherein
the storage unit (106) includes:
a similarity network information storage unit (106b) configured for storing similarity network information on a protein similarity network that includes proteins having similarity, said similarity between proteins being found with a protein signature-based algorithm determining the similarity between two sequence information , said similarity network information being determined as a graph comprising:

- nodes, each node representing a protein, and
- edges, such that two nodes are connected by an edge only if the nodes share considerable protein sequence similarity; and

a drug target storage unit (106c) configured for storing drug information containing approval attributes of drugs on approval or rejection and protein information on the proteins targeted by the drugs in association with each other; and an interaction network information storage unit (106d) configured for storing interaction network information on a protein-protein interaction network that is constructed based on interactions between pre-selected proteins, said interaction between proteins meaning that said proteins are similar, said interaction network information being determined as a graph comprising :

- nodes, each node representing a protein, and
- edges such that two nodes are connected if the corresponding proteins have an interaction; and

the control unit (102) includes:
a similarity centrality measure calculating unit (102b) configured for calculating, based on the similarity network information stored in the similarity network information storage unit (106b), similarity centrality measures that are centrality measures containing:

- a degree centrality, defined as an index representing how much the node is directly connected to nodes in the similarity network,
- a betweenness centrality B(v), using the following expression (1) formed of $S_{ij}$ denoting the number of shortest paths between a node i and a node j, and $S_{ij}(v)$ denoting the fraction of shortest paths passing through a node v:

$$(1) \quad B(v) = \sum \frac{S_{ij}(v)}{S_{ij}} \text{ with } i \neq j, \ v \neq i \text{ and } v \neq j,$$

- a closeness centrality C(v), using the following expression (2) formed of d(v,i) denoting the distance represented at the step between a node v and the node i:

$$(2) \quad C(v) = \frac{1}{\sum d(v,i)} \text{ with } i \neq v \ ,$$

and
- a Burt's constraint C(i) of the proteins included in the protein similarity network, using the following expression (3) formed of $p_{iq}p_{qj}$ denoting a product of the proportional strength of the node j's relationship with the node i and the proportional strength of the node j's relationship with the node q:

$$(3) \quad C(i) = \sum_{j} \left( p_{ij} + \sum_{q} p_{iq}p_{qj} \right)^2 \text{ with } q \neq i, j \text{ and } j \neq i;$$

an approval determining step (SA-2) of, based on the approval attributes of the drugs targeting the proteins according to the protein information stored in the drug target storage unit (106c), which are the proteins that the protein similarity network includes, obtaining during the drug development a determination result representing whether the proteins to be validated, which are proteins that the similarity network includes, are within a range of targets of approved drugs or a range of targets of rejected drugs, using the similarity centrality measures of the proteins to be validated that are calculated at the similarity centrality measure calculating step (SA-1); and
a determination result outputting step (SA-3) of outputting, via the output unit, the determination result that is obtained at the approval determining step (SA-2).

7. A computer program product having a non-transitory tangible computer readable medium including programmed instructions for causing, when executed by an approval prediction apparatus (100) including an output unit, a storage unit (106), and a control unit (102), wherein
the storage unit (106) includes:
a similarity network information storage unit (106b) configured for storing similarity network information on a protein similarity network that is constructed according to the similarity between proteins, said similarity between proteins being found with a protein signature-based algorithm determining the similarity between two sequence information , said similarity network information being determined as a graph comprising:

- nodes, each node representing a protein, and
- edges, such that two nodes are connected by an edge only if the nodes share considerable protein sequence similarity;

a drug target storage unit (106c) configured for storing drug information containing approval attributes of drugs on approval or rejection and protein information on the proteins targeted by the drugs in association with each other; and an interaction network information storage unit (106d) configured for storing interaction network information on a protein-protein interaction network that is constructed based on interactions between pre-determined proteins, said interaction between proteins meaning that said proteins are similar , said interaction network information is being determined as a graph comprising :

- nodes, each node representing a protein, and
- edges such that two nodes are connected if the corresponding proteins have an interaction;

the approval prediction apparatus (100) to perform an approval prediction method comprising:
a similarity centrality measure calculating step (SB-1) of, based on the similarity network information stored in the similarity network information storage unit (106b), calculating similarity centrality measures that are centrality measures containing:

- a degree centrality, defined as an index representing how much the node is directly connected to nodes in the similarity network,
- a betweenness centrality B(v), using the following expression (1) formed of $S_{ij}$ denoting the number of shortest paths between a node i and a node j, and $S_{ij}(v)$ denoting the fraction of shortest paths passing through a node v:

$$(1) \quad B(v) = \sum \frac{S_{ij}(v)}{S_{ij}} \quad \text{with } i \neq j, \; v \neq i \text{ and } v \neq j,$$

- a closeness centrality C(v), using the following expression (2) formed of d(v,i) denoting the distance represented at the step between a node v and the node i:

$$(2) \quad C(v) = \frac{1}{\sum d(v,i)} \quad \text{with } i \neq v \; ,$$

and
- a Burt's constraint C(i) of the proteins that the protein similarity network includes, using the following expression (3) formed of $p_{iq}p_{qj}$ denoting a product of the proportional strength of the node j's relationship with the node i and the proportional strength of the node j's relationship with the node q:

$$(3) \quad C(i) = \sum_j \left( p_{ij} + \sum_q p_{iq}p_{qj} \right)^2 \quad \text{with } q \neq i, j \text{ and } j \neq i$$

an interaction centrality measure calculating step (SB-2) of, based on the interaction network information stored in the interaction network information storage unit (106d), calculating interaction centrality measures that are centrality measures containing said degree centrality, betweenness centrality calculated with expression (1), closeness centrality calculated with expression (2), and Burt's constraint of the proteins that the protein-protein interaction network includes calculated with expression (3);
a rejection score calculating step (SB-3) of calculating during the drug development a rejection score that represents probability of a compound to be validated to be classified as a rejected drug, using classifiers that use, as training

17

data, the approval attributes of the respective drugs stored in the drug target storage unit (106c), the sum and average of the similarity centrality measures per target for each drug that are calculated at the similarity centrality measure calculating step (SB-1), and the sum and average of the interaction centrality measures per target for each drug that are calculated at the interaction centrality measure calculating step (SB-2), said similarity centrality measures used as parameters for several machine learning classifiers; and

a rejection score outputting step (SB-4) of outputting, via the output unit, the rejection score that is calculated at the rejection score calculating step (SB-3).

8. A computer program product having a non-transitory tangible computer readable medium including programmed instructions for causing, when executed by an approval prediction apparatus (100) including an output unit, a storage unit (106), and a control unit (102),

wherein the storage unit (106) includes:

a similarity network information storage unit (106b) configured for storing similarity network information on a protein similarity network that includes proteins having similarity, said similarity between proteins being found with a protein signature-based algorithm determining the similarity between two sequence information, said similarity network information being determined as a graph comprising:

- nodes, each node representing a protein, and
- edges, such that two nodes are connected by an edge only if the nodes share considerable protein sequence similarity; and

a drug target storage unit (106c) configured for storing drug information containing approval attributes of drugs on approval or rejection and protein information on the proteins targeted by the drugs in association with each other; the approval prediction apparatus (100) to perform an approval prediction method comprising:

a similarity centrality measure calculating step (SA-1) of, based on the similarity network information stored in the similarity network information storage unit (106b), calculating similarity centrality measures that are centrality measures containing:

- a degree centrality, defined as an index representing how much the node is directly connected to nodes in the similarity network,
- a betweenness centrality B(v), using the following expression (1) formed of $S_{ij}$ denoting the number of shortest paths between a node i and a node j, and $S_{ij}(v)$ denoting the fraction of shortest paths passing through a node v:

$$(1) \quad B(v) = \sum \frac{S_{ij}(v)}{S_{ij}} \qquad \text{with } i \neq j, \ v \neq i \text{ and } v \neq j,$$

- a closeness centrality C(v), using the following expression (2) formed of d(v,i) denoting the distance represented at the step between a node v and the node i:

$$(2) \quad C(v) = \frac{1}{\sum d(v,i)} \qquad \text{with } i \neq v \ ,$$

and

- a Burt's constraint C(i) of the proteins that the protein similarity network includes, using the following expression (3) formed of $p_{iq}p_{qj}$ denoting a product of the proportional strength of the node j's relationship with the node i and the proportional strength of the node j's relationship with the node q:

$$(3) \quad C(i) = \sum_{j} \left( p_{ij} + \sum_{q} p_{iq} + p_{qj} \right)^{2} \qquad \text{with } q \neq i, j \text{ and } j \neq i \ ;$$

an approval determining step (SA-2) of, based on the approval attributes of the drugs targeting the proteins according to the protein information stored in the drug target storage unit (106c), which are the proteins that the protein similarity network includes, obtaining during the drug development a determination result representing whether the proteins to be validated, which are proteins that the similarity network includes, are within a range of targets of approved drugs or a range of targets of rejected drugs, using the similarity centrality measures of the proteins to be validated that are calculated at the similarity centrality measure calculating step (SA-1); and
a determination result outputting step (SA-3) of outputting, via the output unit, the determination result that is obtained
at the approval determining step (SA-2).

**Patentansprüche**

1. Genehmigungsvorhersagevorrichtung (100) für Medikamentenentwicklung, die eine Ausgabeeinheit, eine Speichereinheit (106) und eine Steuereinheit (102) aufweist, wobei
die Speichereinheit (106) umfasst:
eine Ähnlichkeitsnetzwerkinformationsspeichereinheit (106b), die konfiguriert ist, um Ähnlichkeitsnetzwerkinformationen über ein Proteinähnlichkeitsnetzwerk, das gemäß der Ähnlichkeit zwischen Proteinen aufgebaut ist, zu speichern, wobei die Ähnlichkeit zwischen Proteinen mit einem Proteinsignatur-basierten Algorithmus ermittelt wird, der die Ähnlichkeit zwischen zwei Sequenzinformationen bestimmt, wobei die Ähnlichkeitsnetzwerkinformationen als ein Graph bestimmt werden, der aufweist:

   - Knoten, wobei jeder Knoten ein Protein darstellt, und
   - Kanten, so dass zwei Knoten nur durch eine Kante verbunden sind, wenn die Knoten eine beträchtliche Proteinsequenzähnlichkeit gemeinsam haben;

   eine Medikamentenzielspeichereinheit (106c), die konfiguriert ist, um Medikamenteninformationen, die Genehmigungsattribute von Medikamenten über Genehmigung oder Ablehnung und Proteininformationen über die Proteine, auf welche die Medikamente zielen, in Verbindung miteinander zu speichern; und
   eine Wechselwirkungsnetzwerk-Informationsspeichereinheit (106d), die konfiguriert ist, um Wechselwirkungsnetzwerkinformationen über ein Protein-Protein-Wechselwirkungsnetzwerk zu speichern, das basierend auf Wechselwirkungen zwischen vorausgewählten Proteinen aufgebaut ist, wobei die Wechselwirkung zwischen Proteinen bedeutet, dass die Proteine ähnlich sind, wobei die Wechselwirkungsnetzwerkinformationen als ein Graph bestimmt werden, der aufweist:

   - Knoten, wobei jeder Knoten ein Protein darstellt, und
   - Kanten, so dass zwei Knoten verbunden sind, wenn die entsprechenden Proteine eine Wechselwirkung haben; und

   die Steuereinheit (102) umfasst:
   eine Ähnlichkeitszentralitätsmaß-Berechnungseinheit (102b), die konfiguriert ist, um basierend auf den Ähnlichkeitsnetzwerkinformationen, die in der Ähnlichkeitsnetzwerkinformationsspeichereinheit (106b) gespeichert sind, Ähnlichkeitszentralitätsmaße zu berechnen, welche Zentralitätsmaße sind, die enthalten:

   - einen Grad der Zentralität, der als ein Index definiert ist, der darstellt, wie stark der Knoten direkt mit Knoten in dem Ähnlichkeitsnetzwerk verbunden ist,
   - eine Betweenness-Zentralität B(v), die den folgenden Ausdruck (1) verwendet, der aus $S_{ij}$ gebildet ist, das die Anzahl kürzester Wege zwischen einem Knoten i und einem Knoten j bezeichnet, und wobei $S_{ij}(v)$ den Bruchteil kürzester Wege bezeichnet, die durch einen Knoten v gehen:

$$(1)\ B(v) = \sum \frac{S_{ij}(v)}{S_{ij}} \text{ mit } i \neq j,\ v \neq i \text{ und } v \neq j,$$

   - eine Nähenzentralität C(v), die den folgenden Ausdruck (2) verwendet, der aus d(v, i) gebildet ist, das den Abstand bezeichnet, der in dem Schritt zwischen einem Knoten v und dem Knoten i dargestellt wird:

$$(2)\ C(v) = \frac{1}{\sum d(v,i)} \text{ mit } i \neq v,$$

   und
   - eine Burt-Bedingung C(i) für die Proteine, die in dem Proteinähnlichkeitsnetzwerk enthalten sind, unter Verwendung des folgenden Ausdrucks (3), der aus $p_{iq}p_{qj}$ gebildet wird, das ein Produkt der proportionalen Stärke der Beziehung des Knotens j mit dem Knoten i und der proportionalen Stärke der Beziehung des Knotens j mit dem Knoten q bezeichnet:

$$(3)\ C(i) = \sum_j (p_{ij} + \sum_q p_{iq}\, p_{qj})^2 \text{ mit q} \neq \text{i, j und j} \neq \text{i;}$$

eine Wechselwirkungszentralitätsmaß-Berechnungseinheit (102e), die konfiguriert ist, um basierend auf den Weichselwirkungsnetzwerkinformationen, die in der Wechselwirkungsnetzwerk-Informationsspeichereinheit (106d) gespeichert sind, Wechselwirkungszentralitätsmaße zu berechnen, die Zentralitätsmaße sind, die enthalten: den Grad der Zentralität, die mit dem Ausdruck (1) berechnete Betweenness-Zentralität, die mit dem Ausdruck (2) berechnete Nähenzentralität und die mit dem Ausdruck (3) berechnete Burt-Bedingung der Proteine, die in dem Protein-Protein-Wechselwirkungsnetzwerk enthalten sind;

eine Ablehnungspunktzahlberechnungseinheit (102f), die konfiguriert ist, um während der Medikamentenentwicklung eine Ablehnungspunktzahl, die eine Wahrscheinlichkeit darstellt, dass eine Verbindung, die validiert werden soll, als ein abgelehntes Medikament klassifiziert wird, zu berechnen unter Verwendung von: Klassifikatoren, die als Trainingsdaten die Genehmigungsattribute der jeweiligen Medikamente, die in der Medikamentenzielspeichereinheit (106c) gespeichert sind, verwenden, der Summe und des Mittels der Ähnlichkeitszentralitätsmaße pro Ziel für jedes Medikament, die durch die Ähnlichkeitszentralitätsmaß-Berechnungseinheit (102b) berechnet werden, und der Summe und des Mittels der Wechselwirkungszentralitätsmaße pro Ziel für jedes Medikament, die durch die Wechselwirkungszentralitätsmaß-Berechnungseinheit (102e) berechnet werden, wobei die Ähnlichkeitszentralitätsmaße als Parameter für mehrere Maschinenlernklassifikatoren verwendet werden; und

eine Ablehnungspunktzahlausgabeeinheit (102g), die konfiguriert ist, um die Ablehnungspunktzahl, die durch die Ablehnungspunktzahlberechnungseinheit (102f) berechnet wird, über die Ausgabeeinheit auszugeben.

2. Genehmigungsvorhersagevorrichtung (100) für Medikamentenentwicklung, die eine Ausgabeeinheit, eine Speichereinheit (106) und eine Steuereinheit (102) aufweist, wobei
die Speichereinheit (106) umfasst:
eine Ähnlichkeitsnetzwerkinformationsspeichereinheit (106b), die konfiguriert ist, um Ähnlichkeitsnetzwerkinformationen über ein Proteinähnlichkeitsnetzwerk, das Proteine mit Ähnlichkeit umfasst, zu speichern, wobei die Ähnlichkeit zwischen Proteinen mit einem Proteinsignatur-basierten Algorithmus ermittelt wird, der die Ähnlichkeit zwischen zwei Sequenzinformationen bestimmt, wobei die Ähnlichkeitsnetzwerkinformationen als ein Graph bestimmt werden, der aufweist:

- Knoten, wobei jeder Knoten ein Protein darstellt, und
- Kanten, so dass zwei Knoten nur durch eine Kante verbunden sind, wenn die Knoten eine beträchtliche Proteinsequenzähnlichkeit gemeinsam haben; und

eine Medikamentenzielspeichereinheit (106c), die konfiguriert ist, um Medikamenteninformationen, die Genehmigungsattribute von Medikamenten über Genehmigung oder Ablehnung und Proteininformationen über die Proteine, auf welche die Medikamente zielen, in Verbindung miteinander zu speichern; und
wobei die Steuereinheit (102) umfasst:
eine Ähnlichkeitszentralitätsmaß-Berechnungseinheit (102b), die konfiguriert ist, um basierend auf den Ähnlichkeitsnetzwerkinformationen, die in der Ähnlichkeitsnetzwerkinformationsspeichereinheit (106b) gespeichert sind, Ähnlichkeitszentralitätsmaße zu berechnen, welche Zentralitätsmaße sind, die enthalten:

- einen Grad der Zentralität, der als ein Index definiert ist, der darstellt, wie stark der Knoten direkt mit Knoten in dem Ähnlichkeitsnetzwerk verbunden ist,
- eine Betweenness-Zentralität B(v), die den folgenden Ausdruck (1) verwendet, der aus $S_{ij}$ gebildet ist, das die Anzahl kürzester Wege zwischen einem Knoten i und einem Knoten j bezeichnet, und wobei $S_{ij}(v)$ den Bruchteil kürzester Wege bezeichnet, die durch einen Knoten v gehen:

$$(1)\ B(v) = \sum \frac{S_{ij}(v)}{S_{ij}} \text{ mit i} \neq \text{j, v} \neq \text{i und v} \neq \text{j,}$$

- eine Nähenzentralität C(v), die den folgenden Ausdruck (2) verwendet, der aus d(v, i) gebildet ist, das den Abstand bezeichnet, der in dem Schritt zwischen einem Knoten v und dem Knoten i dargestellt wird:

$$(2)\ C(v) = \frac{1}{\sum d(v,i)}\ \text{mit}\ i \neq v,$$

und
- eine Burt-Bedingung C(i) für die Proteine, die in dem Proteinähnlichkeitsnetzwerk enthalten sind, unter Verwendung des folgenden Ausdrucks (3), der aus $p_{iq}p_{qj}$ gebildet wird, das ein Produkt der proportionalen Stärke der Beziehung des Knotens j mit dem Knoten i und der proportionalen Stärke der Beziehung des Knotens j mit dem Knoten q bezeichnet:

$$(3)\ C(i) = \sum_j (p_{ij} + \sum_q p_{iq}\, p_{qj})^2\ \text{mit}\ q \neq i,\ j\ \text{und}\ j \neq i;$$

eine Genehmigungsbestimmungseinheit (102c), die konfiguriert ist, um während der Medikamentenentwicklung basierend auf den Genehmigungsattributen der Medikamente, die auf die Proteine zielen, gemäß den Proteininformationen, die in der Medikamentenzielspeichereinheit (106c) gespeichert sind, welche die Proteine sind, die in dem Proteinähnlichkeitsnetzwerk enthalten sind, unter Verwendung der Ähnlichkeitszentralitätsmaße der Proteine, die validiert werden sollen, die durch die Ähnlichkeitszentralitätsmaß-Berechnungseinheit (102b) berechnet werden, ein Bestimmungsergebnis zu erhalten, das darstellt, ob die Proteine, die validiert werden sollen, die Proteine sind, welche das Ähnlichkeitsnetzwerk enthält, innerhalb eines Bereichs von Zielen genehmigter Medikamente oder eines Bereichs von Zielen abgelehnter Medikament sind; und
eine Bestimmungsergebnisausgabeeinheit (102d), die konfiguriert ist, um das Bestimmungsergebnis, das durch die Genehmigungsbestimmungseinheit (102c) bestimmt wird, über die Ausgabeeinheit auszugeben.

3. Genehmigungsvorhersagevorrichtung (100) nach Anspruch 1 oder 2, wobei:
die Speichereinheit (106) ferner eine Proteinsequenzinformationsspeichereinheit (106a) umfasst, die konfiguriert ist, um Sequenzinformationen über Aminosäuresequenzen der Proteine zu speichern, und
die Steuereinheit (102) ferner eine Ähnlichkeitsnetzwerkinformationsspeichereinheit (102a) umfasst, die konfiguriert ist, um, wenn unter Verwendung eines Signaturbasierten Algorithmus und basierend auf den Sequenzinformationen, die in der Proteinsequenzinformationsspeichereinheit (106a) gespeichert sind, die Ähnlichkeit zwischen den Proteinen erfasst wird, das Proteinähnlichkeitsnetzwerk zu erzeugen, das die Proteine enthält, zwischen denen die Ähnlichkeit erfasst wird, und um die Ähnlichkeitsnetzwerkinformationen über das Proteinähnlichkeitsnetzwerk in der Ähnlichkeitsnetzwerkinformationsspeichereinheit (106b) zu speichern.

4. Genehmigungsvorhersagevorrichtung (100) nach Anspruch 2, wobei die Genehmigungsbestimmungseinheit (102c) konfiguriert ist, um basierend auf den Genehmigungsattributen der Medikamente, die auf die Proteine zielen, gemäß den Proteininformationen, die in der Medikamentenzielspeichereinheit (106c) gespeichert sind, welche die Proteine sind, die das Proteinähnlichkeitsnetzwerk enthält, ein Bestimmungsergebnis zu erzeugen, das darstellt, dass die Proteine, die validiert werden sollen, innerhalb des Bereichs von Zielen abgelehnter Medikamente enthalten sind, wenn der Grad der Zentralität, der in den Ähnlichkeitszentralitätsmaßen der Proteine, die validiert werden sollen, die durch die Ähnlichkeitszentralitätsmaß-Berechnungseinheit (102b) berechnet werden, hoch ist, die Nähenzentralität niedrig ist und die Burt-Bedingung äußerst niedrig ist.

5. Genehmigungsvorhersageverfahren für Medikamentenentwicklung, das durch eine Genehmigungsvorhersagevorrichtung (100) ausgeführt wird, die eine Ausgabeeinheit, eine Speichereinheit (106) und eine Steuereinheit (102) umfasst, wobei
die Speichereinheit (106) umfasst:
eine Ähnlichkeitsnetzwerkinformationsspeichereinheit (106b), die konfiguriert ist, um Ähnlichkeitsnetzwerkinformationen über ein Proteinähnlichkeitsnetzwerk, das gemäß der Ähnlichkeit zwischen Proteinen aufgebaut ist, zu speichern, wobei die Ähnlichkeit zwischen Proteinen mit einem Proteinsignatur-basierten Algorithmus ermittelt wird, der die Ähnlichkeit zwischen zwei Sequenzinformationen bestimmt, wobei die Ähnlichkeitsnetzwerkinformationen als ein Graph bestimmt werden, der aufweist:

- Knoten, wobei jeder Knoten ein Protein darstellt, und
- Kanten, so dass zwei Knoten nur durch eine Kante verbunden sind, wenn die Knoten eine beträchtliche Proteinsequenzähnlichkeit gemeinsam haben;

eine Medikamentenzielspeichereinheit (106c), die konfiguriert ist, um Medikamenteninformationen, die Genehmigungsattribute von Medikamenten über Genehmigung oder Ablehnung und Proteininformationen über die Proteine, auf welche die Medikamente zielen, in Verbindung miteinander zu speichern; und

eine Wechselwirkungsnetzwerk-Informationsspeichereinheit (106d), die konfiguriert ist, um Wechselwirkungsnetzwerkinformationen über ein Protein-Protein-Wechselwirkungsnetzwerk zu speichern, das basierend auf Wechselwirkungen zwischen vorbestimmten Proteinen aufgebaut ist, wobei die Wechselwirkung zwischen Proteinen bedeutet, dass die Proteine ähnlich sind, wobei die Wechselwirkungsnetzwerkinformationen als ein Graph bestimmt werden, der aufweist:

- Knoten, wobei jeder Knoten ein Protein darstellt, und
- Kanten, so dass zwei Knoten verbunden sind, wenn die entsprechenden Proteine eine Wechselwirkung haben; und

wobei das Verfahren durch die Steuereinheit (102) ausgeführt wird und aufweist:

einen Ähnlichkeitszentralitätsmaß-Berechnungsschritt (SB-1), der basierend auf den Ähnlichkeitsnetzwerkinformationen, die in der Ähnlichkeitsnetzwerkinformationsspeichereinheit (106b) gespeichert sind, Ähnlichkeitszentralitätsmaße berechnet, die Ähnlichkeitszentralitätsmaße sind, die einen Grad der Zentralität, eine mit dem Ausdruck (1) berechnete Betweenness-Zentralität, eine mit dem Ausdruck (2) berechnete Nähenzentralität und eine mit dem Ausdruck (3) berechnete Burt-Bedingung der Proteine, welche das Ähnlichkeitsnetzwerk umfasst, enthalten;

einen Wechselwirkungszentralitätsmaß-Berechnungsschritt (SB-2), der basierend auf den Wechselwirkungsnetzwerkinformationen, die in der Wechselwirkungsnetzwerk-Informationsspeichereinheit (106d) gespeichert sind, Wechselwirkungszentralitätsmaße berechnet, die Zentralitätsmaße sind, die enthalten:

- den Grad der Zentralität, der als ein Index definiert ist, der darstellt, wie stark der Knoten direkt mit Knoten in dem Ähnlichkeitsnetzwerk verbunden ist,
- die Betweenness-Zentralität $B(v)$, die den folgenden Ausdruck (1) verwendet, der aus $S_{ij}$ gebildet ist, das die Anzahl kürzester Wege zwischen einem Knoten i und einem Knoten j bezeichnet, und wobei $S_{ij}(v)$ den Bruchteil kürzester Wege bezeichnet, die durch einen Knoten v gehen:

$$(1)\ B(v) = \sum \frac{S_{ij}(v)}{S_{ij}}\ \text{mit i} \neq \text{j, v} \neq \text{i und v} \neq \text{j},$$

- die Nähenzentralität $C(v)$, die den folgenden Ausdruck (2) verwendet, der aus $d(v, i)$ gebildet ist, das den Abstand bezeichnet, der in dem Schritt zwischen einem Knoten v und dem Knoten i dargestellt wird:

$$(2)\ C(v) = \frac{1}{\sum d(v,i)}\ \text{mit i} \neq \text{v},$$

und

- die Burt-Bedingung $C(i)$ für die Proteine, die in dem Proteinähnlichkeitsnetzwerk enthalten sind, unter Verwendung des folgenden Ausdrucks (3), der aus $p_{iq}p_{qj}$ gebildet wird, das ein Produkt der proportionalen Stärke der Beziehung des Knotens j mit dem Knoten i und der proportionalen Stärke der Beziehung des Knotens j mit dem Knoten q bezeichnet:

$$(3)\ C(i) = \sum_j (p_{ij} + \sum_q p_{iq}\ p_{qj})^2\ \text{mit q} \neq \text{i, j und j} \neq \text{i};$$

einen Ablehnungspunktzahlberechnungsschritt (SB-3), der während der Medikamentenentwicklung eine Ablehnungspunktzahl, die eine Wahrscheinlichkeit darstellt, dass eine Verbindung, die validiert werden soll, als ein abgelehntes Medikament klassifiziert wird, berechnet unter Verwendung von: Klassifikatoren, die als Trainingsdaten die Genehmigungsattribute der jeweiligen Medikamente, die in der Medikamentenzielspeichereinheit (106c) gespeichert sind, verwenden, der Summe und des Mittels der Ähnlichkeitszentralitätsmaße pro Ziel für jedes Medikament, die in dem Ähnlichkeitszentralitätsmaß-Berechnungsschritt (SB-1) berechnet werden, und der Summe und des Mittels der Wechselwirkungszentralitätsmaße pro Ziel für jedes Medikament, die in dem Wechselwirkungszentralitätsmaß-Berechnungsschritt (SB-2) berechnet werden, wobei die Ähnlichkeitszentralitätsmaße als Parameter für mehrere Maschinenlernklassifikatoren verwendet werden; und

einen Ablehnungspunktzahlausgabeschritt (SB-4), der die Ablehnungspunktzahl, die in dem Ablehnungspunktzahlberechnungsschritt (SB-3) berechnet wird, über die Ausgabeeinheit ausgibt.

6. Genehmigungsvorhersageverfahren für Medikamentenentwicklung, die durch eine Genehmigungsvorhersagevorrichtung (100) ausgeführt wird, die eine Ausgabeeinheit, eine Speichereinheit (106) und eine Steuereinheit (102) umfasst, wobei
die Speichereinheit (106) umfasst:
eine Ähnlichkeitsnetzwerkinformationsspeichereinheit (106b), die konfiguriert ist, um Ähnlichkeitsnetzwerkinformationen über ein Proteinähnlichkeitsnetzwerk, das Proteine mit Ähnlichkeit umfasst, zu speichern, wobei die Ähnlichkeit zwischen Proteinen mit einem Proteinsignatur-basierten Algorithmus ermittelt wird, der die Ähnlichkeit zwischen zwei Sequenzinformationen bestimmt, wobei die Ähnlichkeitsnetzwerkinformationen als ein Graph bestimmt werden, der aufweist:

- Knoten, wobei jeder Knoten ein Protein darstellt, und
- Kanten, so dass zwei Knoten nur durch eine Kante verbunden sind, wenn die Knoten eine beträchtliche Proteinsequenzähnlichkeit gemeinsam haben; und

eine Medikamentenzielspeichereinheit (106c), die konfiguriert ist, um Medikamenteninformationen, die Genehmigungsattribute von Medikamenten über Genehmigung oder Ablehnung und Proteininformationen über die Proteine, auf welche die Medikamente zielen, in Verbindung miteinander zu speichern; und
eine Wechselwirkungsnetzwerk-Informationsspeichereinheit (106d), die konfiguriert ist, um Wechselwirkungsnetzwerkinformationen über ein Protein-Protein-Wechselwirkungsnetzwerk zu speichern, das basierend auf Wechselwirkungen zwischen vorbestimmten Proteinen aufgebaut ist, wobei die Wechselwirkung zwischen Proteinen bedeutet, dass die Proteine ähnlich sind, wobei die Wechselwirkungsnetzwerkinformationen als ein Graph bestimmt werden, der aufweist:

- Knoten, wobei jeder Knoten ein Protein darstellt, und
- Kanten, so dass zwei Knoten verbunden sind, wenn die entsprechenden Proteine eine Wechselwirkung haben;

wobei das Verfahren durch die Steuereinheit (102) ausgeführt wird und aufweist:

einen Ähnlichkeitszentralitätsmaß-Berechnungsschritt (SB-1), der basierend auf den Ähnlichkeitsnetzwerkinformationen, die in der Ähnlichkeitsnetzwerkinformationsspeichereinheit (106b) gespeichert sind, Ähnlichkeitszentralitätsmaße berechnet, welche Zentralitätsmaße sind, die einen Grad der Zentralität, eine mit dem Ausdruck (1) berechnete Betweenness-Zentralität, eine mit dem Ausdruck (2) berechnete Nähenzentralität und eine mit dem Ausdruck (3) berechnete Burt-Bedingung der Proteine, welche das Ähnlichkeitsnetzwerk umfasst, enthalten;
einen Wechselwirkungszentralitätsmaß-Berechnungsschritt (SB-2), der basierend auf Wechselwirkungsnetzwerkinformationen, die in der Wechselwirkungsnetzwerk-Informationsspeichereinheit (106d) gespeichert sind, Wechselwirkungszentralitätsmaße berechnet, die Zentralitätsmaße sind, die enthalten:

- den Grad der Zentralität, der als ein Index definiert ist, der darstellt, wie stark der Knoten direkt mit Knoten in dem Ähnlichkeitsnetzwerk verbunden ist,
- die Betweenness-Zentralität B(v), die den folgenden Ausdruck (1) verwendet, der aus $S_{ij}$ gebildet ist, das die Anzahl kürzester Wege zwischen einem Knoten i und einem Knoten j bezeichnet, und wobei $S_{ij}(v)$ den Bruchteil kürzester Wege bezeichnet, die durch einen Knoten v gehen:

$$(1)\ B(v) = \sum \frac{S_{ij}(v)}{S_{ij}} \text{ mit } i \neq j,\ v \neq i \text{ und } v \neq j,$$

- die Nähenzentralität C(v), die den folgenden Ausdruck (2) verwendet, der aus d(v, i) gebildet ist, das den Abstand bezeichnet, der in dem Schritt zwischen einem Knoten v und dem Knoten i dargestellt wird:

$$(2)\ C(v) = \frac{1}{\sum d(v,i)}\ \text{mit i} \neq \text{v},$$

und
- die Burt-Bedingung C(i) für die Proteine, die in dem Proteinähnlichkeitsnetzwerk enthalten sind, unter Verwendung des folgenden Ausdrucks (3), der aus $p_{iq}p_{qj}$ gebildet wird, das ein Produkt der proportionalen Stärke der Beziehung des Knotens j mit dem Knoten i und der proportionalen Stärke der Beziehung des Knotens j mit dem Knoten q bezeichnet:

$$(3)\ C(i) = \sum_j (p_{ij} + \sum_q p_{iq}\, p_{qj})^2\ \text{mit q} \neq \text{i, j und j} \neq \text{i};$$

einen Genehmigungsbestimmungsschritt (SA-2), der während der Medikamentenentwicklung basierend auf den Genehmigungsattributen der Medikamente, die auf die Proteine zielen, gemäß den Proteininformationen, die in der Medikamentenzielspeichereinheit (106c) gespeichert sind, welche die Proteine sind, die in dem Proteinähnlichkeitsnetzwerk enthalten sind, unter Verwendung der Ähnlichkeitszentralitätsmaße der Proteine, die validiert werden sollen, die in dem Ähnlichkeitszentralitätsmaß-Berechnungsschritt (SA-1) berechnet werden, ein Bestimmungsergebnis erhält, das darstellt, ob die Proteine, die validiert werden sollen, die Proteine sind, welche das Ähnlichkeitsnetzwerk enthält, innerhalb eines Bereichs von Zielen genehmigter Medikamente oder eines Bereichs von Zielen abgelehnter Medikament sind; und
einen Bestimmungsergebnisausgabeschritt (SA-3), der das Bestimmungsergebnis, das in dem Genehmigungsbestimmungsschritt (SA-2) bestimmt wird, über die Ausgabeeinheit ausgibt.

7. Computerprogrammprodukt mit einem nicht flüchtigen materiellen computerlesbaren Medium, das programmierte Anweisungen umfasst, um, wenn es von einer Genehmigungsvorhersagevorrichtung (100) ausgeführt wird, die eine Ausgabeeinheit, eine Speichereinheit (106) und eine Steuereinheit (102) umfasst, zu bewirken, wobei
die Speichereinheit (106) umfasst:
eine Ähnlichkeitsnetzwerkinformationsspeichereinheit (106b), die konfiguriert ist, um Ähnlichkeitsnetzwerkinformationen über ein Proteinähnlichkeitsnetzwerk, das gemäß der Ähnlichkeit zwischen Proteinen aufgebaut ist, zu speichern, wobei die Ähnlichkeit zwischen Proteinen mit einem Proteinsignatur-basierten Algorithmus ermittelt wird, der die Ähnlichkeit zwischen zwei Sequenzinformationen bestimmt, wobei die Ähnlichkeitsnetzwerkinformationen als ein Graph bestimmt werden, der aufweist:

- Knoten, wobei jeder Knoten ein Protein darstellt, und
- Kanten, so dass zwei Knoten nur durch eine Kante verbunden sind, wenn die Knoten eine beträchtliche Proteinsequenzähnlichkeit gemeinsam haben;

eine Medikamentenzielspeichereinheit (106c), die konfiguriert ist, um Medikamenteninformationen, die Genehmigungsattribute von Medikamenten über Genehmigung oder Ablehnung und Proteininformationen über die Proteine, auf welche die Medikamente zielen, in Verbindung miteinander zu speichern; und
eine Wechselwirkungsnetzwerk-Informationsspeichereinheit (106d), die konfiguriert ist, um Wechselwirkungsnetzwerkinformationen über ein Protein-Protein-Wechselwirkungsnetzwerk zu speichern, das basierend auf Wechselwirkungen zwischen vorbestimmten Proteinen aufgebaut ist, wobei die Wechselwirkung zwischen Proteinen bedeutet, dass die Proteine ähnlich sind, wobei die Wechselwirkungsnetzwerkinformationen als ein Graph bestimmt werden, der aufweist:

- Knoten, wobei jeder Knoten ein Protein darstellt, und
- Kanten, so dass zwei Knoten verbunden sind, wenn die entsprechenden Proteine eine Wechselwirkung haben;

die Genehmigungsvorhersagevorrichtung (100) ein Genehmigungsvorhersageverfahren durchführen soll, das aufweist:
einen Ähnlichkeitszentralitätsmaß-Berechnungsschritt (SB-1), der basierend auf den Ähnlichkeitsnetzwerkinformationen, die in der Ähnlichkeitsnetzwerkinformationsspeichereinheit (106b) gespeichert sind, Ähnlichkeitszentralitätsmaße berechnet, die Zentralitätsmaße sind, die enthalten:

- einen Grad der Zentralität, der als ein Index definiert ist, der darstellt, wie stark der Knoten direkt mit Knoten in dem Ähnlichkeitsnetzwerk verbunden ist,
- eine Betweenness-Zentralität B(v), die den folgenden Ausdruck (1) verwendet, der aus $S_{ij}$ gebildet ist, das die Anzahl kürzester Wege zwischen einem Knoten i und einem Knoten j bezeichnet, und wobei $S_{ij}(v)$ den Bruchteil kürzester Wege bezeichnet, die durch einen Knoten v gehen:

$$(1)\ B(v) = \sum \frac{S_{ij}(v)}{S_{ij}} \text{ mit i} \neq \text{j, v} \neq \text{i und v} \neq \text{j,}$$

- eine Nähenzentralität C(v), die den folgenden Ausdruck (2) verwendet, der aus d(v, i) gebildet ist, das den Abstand bezeichnet, der in dem Schritt zwischen einem Knoten v und dem Knoten i dargestellt wird:

$$(2)\ C(v) = \frac{1}{\sum d(v,i)} \text{ mit i} \neq \text{v,}$$

und
- eine Burt-Bedingung C(i) für die Proteine, die in dem Proteinähnlichkeitsnetzwerk enthalten sind, unter Verwendung des folgenden Ausdrucks (3), der aus $p_{iq}p_{qj}$ gebildet wird, das ein Produkt der proportionalen Stärke der Beziehung des Knotens j mit dem Knoten i und der proportionalen Stärke der Beziehung des Knotens j mit dem Knoten q bezeichnet:

$$(3)\ C(i) = \sum_j (p_{ij} + \sum_q p_{iq}\ p_{qj})^2 \text{ mit q} \neq \text{i, j und j} \neq \text{i;}$$

einen Wechselwirkungszentralitätsmaß-Berechnungsschritt (SB-2), der basierend auf Wechselwirkungsnetzwerkinformationen, die in der Wechselwirkungsnetzwerk-Informationsspeichereinheit (106d) gespeichert sind, Wechselwirkungszentralitätsmaße berechnet, die Zentralitätsmaße sind, die den Grad der Zentralität, die mit dem Ausdruck (1) berechnete Betweenness-Zentralität, die mit dem Ausdruck (2) berechnete Nähenzentralität und die mit dem Ausdruck (3) berechnete Burt-Bedingung der Proteine, welche das Ähnlichkeitsnetzwerk umfasst, enthalten;
einen Ablehnungspunktzahlberechnungsschritt (SB-3), der während der Medikamentenentwicklung eine Ablehnungspunktzahl, die eine Wahrscheinlichkeit darstellt, dass eine Verbindung, die validiert werden soll, als ein abgelehntes Medikament klassifiziert wird, berechnet unter Verwendung von: Klassifikatoren, die als Trainingsdaten die Genehmigungsattribute der jeweiligen Medikamente, die in der Medikamentenzielspeichereinheit (106c) gespeichert sind, verwenden, der Summe und des Mittels der Ähnlichkeitszentralitätsmaße pro Ziel für jedes Medikament, die in dem Ähnlichkeitszentralitätsmaß-Berechnungsschritt (SB-1) berechnet werden, und der Summe und des Mittels der Wechselwirkungszentralitätsmaße pro Ziel für jedes Medikament, die in dem Wechselwirkungszentralitätsmaß-Berechnungsschritt (SB-2) berechnet werden, wobei die Ähnlichkeitszentralitätsmaße als Parameter für mehrere Maschinenlernklassifikatoren verwendet werden; und
einen Ablehnungspunktzahlausgabeschritt (SB-4), der die Ablehnungspunktzahl, die in dem Ablehnungspunktzahlberechnungsschritt (SB-3) berechnet wird, über die Ausgabeeinheit ausgibt.

8. Computerprogrammprodukt mit einem nicht flüchtigen materiellen computerlesbaren Medium, das programmierte Anweisungen umfasst, um, wenn es von einer Genehmigungsvorhersagevorrichtung (100) ausgeführt wird, die eine Ausgabeeinheit, eine Speichereinheit (106) und eine Steuereinheit (102) umfasst, zu bewirken, wobei
die Speichereinheit (106) umfasst:
eine Ähnlichkeitsnetzwerkinformationsspeichereinheit (106b), die konfiguriert ist, um Ähnlichkeitsnetzwerkinformationen über ein Proteinähnlichkeitsnetzwerk, das Proteine mit Ähnlichkeit umfasst, zu speichern, wobei die Ähnlichkeit zwischen Proteinen mit einem Proteinsignatur-basierten Algorithmus ermittelt wird, der die Ähnlichkeit zwischen zwei Sequenzinformationen bestimmt, wobei die Ähnlichkeitsnetzwerkinformationen als ein Graph bestimmt werden, der aufweist:

- Knoten, wobei jeder Knoten ein Protein darstellt, und
- Kanten, so dass zwei Knoten nur durch eine Kante verbunden sind, wenn die Knoten eine beträchtliche Proteinsequenzähnlichkeit gemeinsam haben; und

eine Medikamentenzielspeichereinheit (106c), die konfiguriert ist, um Medikamenteninformationen, die Genehmigungsattribute von Medikamenten über Genehmigung oder Ablehnung und Proteininformationen über die Proteine, auf welche die Medikamente zielen, in Verbindung miteinander zu speichern;

die Genehmigungsvorhersagevorrichtung (100) ein Genehmigungsvorhersageverfahren durchführen soll, das aufweist:

einen Ähnlichkeitszentralitätsmaß-Berechnungsschritt (SA-1), der basierend auf den Ähnlichkeitsnetzwerkinformationen, die in der Ähnlichkeitsnetzwerkinformationsspeichereinheit (106b) gespeichert sind, Ähnlichkeitszentralitätsmaße berechnet, die Zentralitätsmaße sind, die enthalten:

- einen Grad der Zentralität, der als ein Index definiert ist, der darstellt, wie stark der Knoten direkt mit Knoten in dem Ähnlichkeitsnetzwerk verbunden ist,
- eine Betweenness-Zentralität B(v), die den folgenden Ausdruck (1) verwendet, der aus $S_{ij}$ gebildet ist, das die Anzahl kürzester Wege zwischen einem Knoten i und einem Knoten j bezeichnet, und wobei $S_{ij}(v)$ den Bruchteil kürzester Wege bezeichnet, die durch einen Knoten v gehen:

$$(1)\ B(v) = \sum \frac{S_{ij}(v)}{S_{ij}} \text{ mit i} \neq \text{j, v} \neq \text{i und v} \neq \text{j,}$$

- eine Nähenzentralität C(v), die den folgenden Ausdruck (2) verwendet, der aus d(v, i) gebildet ist, das den Abstand bezeichnet, der in dem Schritt zwischen einem Knoten v und dem Knoten i dargestellt wird:

$$(2)\ C(v) = \frac{1}{\sum d(v,i)} \text{ mit i} \neq \text{v,}$$

und
- eine Burt-Bedingung C(i) für die Proteine, die in dem Proteinähnlichkeitsnetzwerk enthalten sind, unter Verwendung des folgenden Ausdrucks (3), der aus $p_{iq}p_{qj}$ gebildet wird, das ein Produkt der proportionalen Stärke der Beziehung des Knotens j mit dem Knoten i und der proportionalen Stärke der Beziehung des Knotens j mit dem Knoten q bezeichnet:

$$(3)\ C(i) = \sum_j (p_{ij} + \sum_q p_{iq}\, p_{qj})^2 \text{ mit q} \neq \text{i, j und j} \neq \text{i;}$$

einen Genehmigungsbestimmungsschritt (SA-2), der während der Medikamentenentwicklung basierend auf den Genehmigungsattributen der Medikamente, die auf die Proteine zielen, gemäß den Proteininformationen, die in der Medikamentenzielspeichereinheit (106c) gespeichert sind, welche die Proteine sind, die in dem Proteinähnlichkeitsnetzwerk enthalten sind, unter Verwendung der Ähnlichkeitszentralitätsmaße der Proteine, die validiert werden sollen, die in dem Ähnlichkeitszentralitätsmaß-Berechnungsschritt (SA-1) berechnet werden, ein Bestimmungsergebnis erhält, das darstellt, ob die Proteine, die validiert werden sollen, die Proteine sind, welche das Ähnlichkeitsnetzwerk enthält, innerhalb eines Bereichs von Zielen genehmigter Medikamente oder eines Bereichs von Zielen abgelehnter Medikament sind; und

einen Bestimmungsergebnisausgabeschritt (SA-3), der das Bestimmungsergebnis, das in dem Genehmigungsbestimmungsschritt (SA-2) bestimmt wird, über die Ausgabeeinheit ausgibt.

**Revendications**

1. Appareil de prédiction d'approbation (100) pour un développement de médicament comprenant une unité de sortie, une unité de stockage (106), et une unité de commande (102), dans lequel
l'unité de stockage (106) inclut :
une unité de stockage d'informations de réseau de similarité (106b) configurée pour stocker des informations de réseau de similarité sur un réseau de similarité de protéine qui est construit en fonction de la similarité entre protéines, ladite similarité entre protéines étant trouvée avec un algorithme basé sur une signature de protéine déterminant la similarité entre deux informations de séquence, lesdites informations de réseau de similarité étant déterminées comme un graphe comprenant :

- des nœuds, chaque nœud représentant une protéine, et
- des arêtes, de sorte que deux nœuds ne soient reliés par une arête que si les nœuds partagent une similarité de séquence de protéine considérable ;

une unité de stockage de cible de médicament (106c) configurée pour stocker des informations de médicament contenant des attributs d'approbation de médicaments sur l'approbation ou le rejet et des informations de protéine sur les protéines ciblées par les médicaments en association les unes avec les autres ; et
une unité de stockage d'informations de réseau d'interaction (106d) configurée pour stocker des informations de réseau d'interaction sur un réseau d'interaction protéine-protéine qui est construit sur la base d'interactions entre des protéines présélectionnées, ladite interaction entre protéines signifiant que lesdites protéines sont similaires, lesdites informations de réseau d'interaction étant déterminées comme un graphe comprenant :

- des nœuds, chaque nœud représentant une protéine, et
- des arêtes de sorte que deux nœuds soient reliés si les protéines correspondantes ont une interaction ; et

l'unité de commande (102) inclut :
une unité de calcul de mesure de centralité de similarité (102b) configurée pour calculer, sur la base des informations de réseau de similarité stockées dans l'unité de stockage d'informations de réseau de similarité (106b), des mesures de centralité de similarité qui sont des mesures de centralité contenant :

- une centralité de degré, définie comme un indice représentant le degré auquel le nœud est relié directement à des nœuds dans le réseau de similarité,
- une centralité d'intermédiarité B(v), utilisant l'expression (1) suivante formée de $S_{ij}$ désignant le nombre de chemins les plus courts entre un nœud i et un nœud j, et $S_{ij}(v)$ désignant la fraction de chemins les plus courts traversant un nœud v :

$$(1) \ \mathrm{B(v)} = \sum \frac{S_{ij}(v)}{S_{ij}} \ \text{avec } i \neq j, \ v \neq i \text{ et } v \neq j,$$

- une centralité de proximité C(v), utilisant l'expression (2) suivante formée de d(v, i) désignant la distance représentée à l'étape entre un nœud v et le nœud i :

$$(2) \ \mathrm{C(v)} = \frac{1}{\sum d(v,i)} \ \text{avec } i \neq v,$$

et
- une contrainte de Burt C(i) des protéines incluses dans le réseau de similarité de protéine, utilisant l'expression (3) suivante formée de $p_{iq}p_{qj}$ désignant un produit de l'intensité proportionnelle de la relation du nœud j avec le nœud i et de l'intensité proportionnelle de la relation du nœud j avec le nœud q :

$$(3) \ \mathrm{C(i)} = \sum_{j} \left( p_{ij} + \sum_{q} p_{iq}p_{qj} \right)^2 \ \text{avec } q \neq i, \ j \text{ et } j \neq i \ ;$$

une unité de calcul de mesure de centralité d'interaction (102e) configurée pour calculer, sur la base des informations de réseau d'interaction stockées dans l'unité de stockage d'informations de réseau d'interaction (106d), des mesures de centralité d'interaction qui sont des mesures de centralité contenant lesdites centralité de degré, centralité d'intermédiarité calculée avec l'expression (1), centralité de proximité calculée avec l'expression (2), et contrainte de Burt des protéines incluses dans le réseau d'interaction protéine-protéine calculée avec l'expression (3) ;
une unité de calcul de score de rejet (102f) configurée pour calculer pendant le développement de médicament un score de rejet qui représente une probabilité qu'un composé à valider soit classifié comme un médicament rejeté, en utilisant des classificateurs qui utilisent, en tant que données d'apprentissage, les attributs d'approbation des médicaments respectifs stockés dans l'unité de stockage de cible de médicament (106c), la somme et la moyenne des mesures de centralité de similarité par cible pour chaque médicament qui sont calculées par l'unité de calcul de mesure de centralité de similarité (102b), et la somme et la moyenne des mesures de centralité d'interaction par cible pour chaque médicament qui sont calculées par l'unité de calcul de mesure de centralité d'interaction (102e), lesdites mesures de centralité de similarité utilisées en tant que paramètres pour plusieurs classificateurs d'apprentissage machine ; et

une unité de sortie de score de rejet (102g) configurée pour sortir, via l'unité de sortie, le score de rejet qui est calculé par l'unité de calcul de score de rejet (102f).

**2.** Appareil de prédiction d'approbation (100) pour un développement de médicament comprenant une unité de sortie, une unité de stockage (106), et une unité de commande (102), dans lequel

l'unité de stockage (106) inclut :

une unité de stockage d'informations de réseau de similarité (106b) configurée pour stocker des informations de réseau de similarité sur un réseau de similarité de protéine qui inclut des protéines ayant une similarité, ladite similarité entre protéines étant trouvée avec un algorithme basé sur une signature de protéine déterminant la similarité entre deux informations de séquence, lesdites informations de réseau de similarité étant déterminées comme un graphe comprenant :

- des nœuds, chaque nœud représentant une protéine, et
- des arêtes, de sorte que deux nœuds ne soient reliés par une arête que si les nœuds partagent une similarité de séquence de protéine considérable ; et

une unité de stockage de cible de médicament (106c) configurée pour stocker des informations de médicament contenant des attributs d'approbation de médicaments sur l'approbation ou le rejet et des informations de protéine sur les protéines ciblées par les médicaments en association les unes avec les autres ; et

dans lequel l'unité de commande (102) inclut :

une unité de calcul de mesure de centralité de similarité (102b) configurée pour calculer, sur la base des informations de réseau de similarité stockées dans l'unité de stockage d'informations de réseau de similarité (106b), des mesures de centralité de similarité qui sont des mesures de centralité contenant :

- une centralité de degré, définie comme un indice représentant le degré auquel le nœud est relié directement à des nœuds dans le réseau de similarité,
- une centralité d'intermédiarité B(v), utilisant l'expression (1) suivante formée de $S_{ij}$ désignant le nombre de chemins les plus courts entre un nœud i et un nœud j, et $S_{ij}(v)$ désignant la fraction de chemins les plus courts traversant un nœud v :

$$(1) \ B(v) = \sum \frac{S_{ij}(v)}{S_{ij}} \text{ avec } i \neq j, \ v \neq i \text{ et } v \neq j,$$

- une centralité de proximité C(v), utilisant l'expression (2) suivante formée de d(v, i) désignant la distance représentée à l'étape entre un nœud v et le nœud i :

$$(2) \ C(v) = \frac{1}{\sum d(v,i)} \text{ avec } i \neq v,$$

et
- une contrainte de Burt C(i) des protéines que le réseau de similarité de protéine inclut, utilisant l'expression (3) suivante formée de $p_{iq}p_{qj}$ désignant un produit de l'intensité proportionnelle de la relation du nœud j avec le nœud i et de l'intensité proportionnelle de la relation du nœud j avec le nœud q :

$$C(i) = \sum_{j} \left( p_{ij} + \sum_{q} p_{iq}p_{qj} \right)^2 \text{ avec } q \neq i, \ j \text{ et } j \neq i \ ;$$

une unité de détermination d'approbation (102c) configurée pour obtenir, sur la base des attributs d'approbation des médicaments ciblant les protéines en fonction des informations de protéine stockées dans l'unité de stockage de cible de médicament (106c), qui sont les protéines incluses dans le réseau de similarité de protéine, pendant le développement de médicament un résultat de détermination représentant si les protéines à valider, qui sont des protéines que le réseau de similarité inclut, se situent dans une plage de cibles de médicaments approuvés ou une plage de cibles de médicaments rejetés, en utilisant les mesures de centralité de similarité des protéines à valider qui sont calculées par l'unité de calcul de mesure de centralité de similarité (102b) ; et
une unité de sortie de résultat de détermination (102d) configurée pour sortir, via l'unité de sortie, le résultat de détermination qui est obtenu par l'unité de détermination d'approbation (102c).

3. Appareil de prédiction d'approbation (100) selon la revendication 1 ou 2, dans lequel
l'unité de stockage (106) inclut en outre une unité de stockage d'informations de séquence de protéine (106a) configuré pour stocker des informations de séquence sur des séquences d'acides aminés des protéines, et
l'unité de commande (102) inclut en outre une unité de stockage d'informations de réseau de similarité (102a) configurée pour créer, lorsque la similarité est détectée entre les protéines en utilisant un algorithme basé sur une signature et sur la base des informations de séquence stockées dans l'unité de stockage d'informations de séquence de protéine (106a), le réseau de similarité de protéine incluant les protéines entre lesquelles la similarité est détecté et pour stocker les informations de réseau de similarité sur le réseau de similarité de protéine dans l'unité de stockage d'informations de réseau de similarité (106b).

4. Appareil de prédiction d'approbation (100) selon la revendication 2, dans lequel, sur la base des attributs d'approbation des médicaments ciblant les protéines en fonction des informations de protéine stockées dans l'unité de stockage de cible de médicament (106c), qui sont les protéines que le réseau de similarité de protéine inclut, l'unité de détermination d'approbation (102c) est configurée pour générer un résultat de détermination représentant le fait que les protéines à valider se situent dans la plage de cibles de médicaments rejetés lorsque la centralité de degré contenue dans les mesures de centralité de similarité des protéines à valider qui sont calculées par l'unité de calcul de la mesure de centralité de similarité (102b) est élevée, la centralité de proximité est faible, et la contrainte de Burt est extrêmement faible.

5. Procédé de prédiction d'approbation pour un développement de médicament exécuté par un appareil de prédiction d'approbation (100) incluant une unité de sortie, une unité de stockage (106), et une unité de commande (102), dans lequel l'unité de stockage (106) inclut :

une unité de stockage d'informations de réseau de similarité (106b) configurée pour stocker des informations de réseau de similarité sur un réseau de similarité de protéine qui est construit en fonction de la similarité entre protéines, ladite similarité entre protéines étant trouvée avec un algorithme basé sur une signature de protéine déterminant la similarité entre deux informations de séquence, lesdites informations de réseau de similarité étant déterminées comme un graphe comprenant :

- des nœuds, chaque nœud représentant une protéine, et
- des arêtes, de sorte que deux nœuds ne soient reliés par une arête que si les nœuds partagent une similarité de séquence de protéine considérable ;

une unité de stockage de cible de médicament (106c) configurée pour stocker des informations de médicament contenant des attributs d'approbation de médicaments sur l'approbation ou le rejet et des informations de protéine sur les protéines ciblées par les médicaments en association les unes avec les autres ; et
une unité de stockage d'informations de réseau d'interaction (106d) configurée pour stocker des informations de réseau d'interaction sur un réseau d'interaction protéine-protéine qui est construit sur la base d'interactions entre des protéines prédéterminées, ladite interaction entre protéines signifiant que lesdites protéines sont similaires, lesdites informations de réseau d'interaction étant déterminées comme un graphe comprenant :

- des nœuds, chaque nœud représentant une protéine, et
- des arêtes de sorte que deux nœuds soient reliés si les protéines correspondantes ont une interaction ;

le procédé exécuté par l'unité de commande (102) comprenant :

une étape de calcul de mesure de centralité de similarité (SB-1) consistant à, sur la base des informations de réseau de similarité stockées dans l'unité de stockage d'informations de réseau de similarité (106b), calculer des mesures de centralité de similarité qui sont des mesures de centralité contenant une centralité de degré, une centralité d'intermédiarité calculée avec l'expression (1), une centralité de proximité calculée avec l'expression (2), et une contrainte de Burt des protéines que le réseau de similarité de protéine inclut calculée avec l'expression (3) ;
une étape de calcul de mesure de centralité d'interaction (SB-2) consistant à, sur la base des informations de réseau d'interaction stockées dans l'unité de stockage d'informations de réseau d'interaction (106d), calculer des mesures de centralité d'interaction qui sont des mesures de centralité contenant :

- la centralité de degré, définie comme un indice représentant le degré auquel le nœud est relié directement à des nœuds dans le réseau de similarité,
- la centralité d'intermédiarité B(v), utilisant l'expression (1) suivante formée de $S_{ij}$ désignant le nombre de chemins les plus courts entre un nœud i et un nœud j, et $S_{ij}(v)$ désignant la fraction de chemins les plus courts traversant un nœud v :

$$(1)\ B(v) = \sum \frac{S_{ij}(v)}{S_{ij}} \text{ avec } i \neq j,\ v \neq i \text{ et } v \neq j,$$

- la centralité de proximité C(v), utilisant l'expression (2) suivante formée de d(v, i) désignant la distance représentée à l'étape entre un nœud v et le nœud i :

$$(2)\ C(v) = \frac{1}{\sum d(v,i)} \text{ avec } i \neq v,$$

et
- la contrainte de Burt C(i) des protéines que le réseau de similarité de protéine inclut, utilisant l'expression (3) suivante formée de $p_{iq}p_{qj}$ désignant un produit de l'intensité proportionnelle de la relation du nœud j avec le nœud i et de l'intensité proportionnelle de la relation du nœud j avec le nœud q :

$$(3)\ C(i) = \sum_j (p_{ij} + \sum_q p_{iq} p_{qj})^2 \text{ avec } q \neq i,\ j \text{ et } j \neq i\ ;$$

une étape de calcul de score de rejet (SB-3) consistant à calculer pendant le développement de médicament un score de rejet qui représente une probabilité qu'un composé à valider soit classifié comme un médicament rejeté, en utilisant des classificateurs qui utilisent, en tant que données d'apprentissage, les attributs d'approbation des médicaments respectifs stockés dans l'unité de stockage de cible de médicament (106c), la somme et la moyenne des mesures de centralité de similarité par cible pour chaque médicament qui sont calculées à l'étape de calcul de mesure de centralité de similarité (SB-1), et la somme et la moyenne des mesures de centralité d'interaction par cible pour chaque médicament qui sont calculées à l'étape de calcul de mesure de centralité d'interaction (SB-2), lesdites mesures de centralité de similarité utilisées en tant que paramètres pour plusieurs classificateurs d'apprentissage machine ; et
une étape de sortie de score de rejet (SB-4) consistant à sortir, via l'unité de sortie, le score de rejet qui est calculé à l'étape de calcul de score de rejet (SB-3).

6. Procédé de prédiction d'approbation pour un développement de médicament exécuté par un appareil de prédiction d'approbation (100) incluant une unité de sortie, une unité de stockage (106), et une unité de commande (102), dans lequel l'unité de stockage (106) inclut :

une unité de stockage d'informations de réseau de similarité (106b) configurée pour stocker des informations de réseau de similarité sur un réseau de similarité de protéine qui inclut des protéines ayant une similarité, ladite similarité entre protéines étant trouvée avec un algorithme basé sur une signature de protéine déterminant la similarité entre deux informations de séquence, lesdites informations de réseau de similarité étant déterminées comme un graphe comprenant :

- des nœuds, chaque nœud représentant une protéine, et
- des arêtes, de sorte que deux nœuds ne soient reliés par une arête que si les nœuds partagent une similarité de séquence de protéine considérable ; et

une unité de stockage de cible de médicament (106c) configurée pour stocker des informations de médicament contenant des attributs d'approbation de médicaments sur l'approbation ou le rejet et des informations de protéine sur les protéines ciblées par les médicaments en association les unes avec les autres ; et
une unité de stockage d'informations de réseau d'interaction (106d) configurée pour stocker des informations

EP 2 905 363 B1

de réseau d'interaction sur un réseau d'interaction protéine-protéine qui est construit sur la base d'interactions entre des protéines prédéterminées, ladite interaction entre protéines signifiant que lesdites protéines sont similaires, lesdites informations de réseau d'interaction étant déterminées comme un graphe comprenant :

- des nœuds, chaque nœud représentant une protéine, et
- des arêtes de sorte que deux nœuds soient reliés si les protéines correspondantes ont une interaction ; et

le procédé exécuté par l'unité de commande (102) comprenant :

une étape de calcul de mesure de centralité de similarité (SB-1) consistant à, sur la base des informations de réseau de similarité stockées dans l'unité de stockage d'informations de réseau de similarité (106b), calculer des mesures de centralité de similarité qui sont des mesures de centralité contenant une centralité de degré, une centralité d'intermédiarité calculée avec l'expression (1), une centralité de proximité calculée avec l'expression (2), et une contrainte de Burt des protéines que le réseau de similarité de protéine inclut calculée avec l'expression (3) ;
une étape de calcul de mesure de centralité d'interaction (SB-2) consistant à, sur la base des informations de réseau d'interaction stockées dans l'unité de stockage d'informations de réseau d'interaction (106d), calculer des mesures de centralité d'interaction qui sont des mesures de centralité contenant :

- la centralité de degré, définie comme un indice représentant le degré auquel le nœud est relié directement à des nœuds dans le réseau de similarité,
- la centralité d'intermédiarité B(v), utilisant l'expression (1) suivante formée de $S_{ij}$ désignant le nombre de chemins les plus courts entre un nœud i et un nœud j, et $S_{ij}(v)$ désignant la fraction de chemins les plus courts traversant un nœud v :

$$(1)\ B(v) = \sum \frac{S_{ij}(v)}{S_{ij}} \text{ avec } i \neq j, v \neq i \text{ et } v \neq j,$$

- la centralité de proximité C(v), utilisant l'expression (2) suivante formée de d(v, i) désignant la distance représentée à l'étape entre un nœud v et le nœud i :

$$(2)\ C(v) = \frac{1}{\sum d(v,i)} \text{ avec } i \neq v,$$

et
- la contrainte de Burt C(i) des protéines que le réseau de similarité de protéine inclut, utilisant l'expression (3) suivante formée de $p_{iq}p_{qj}$ désignant un produit de l'intensité proportionnelle de la relation du nœud j avec le nœud i et de l'intensité proportionnelle de la relation du nœud j avec le nœud q :

$$(3)\ C(i) = \sum_{j} \left( p_{ij} + \sum_{q} p_{iq}p_{qj} \right)^2 \text{ avec } q \neq i, j \text{ et } j \neq i ;$$

une étape de détermination d'approbation (SA-2) consistant à, sur la base des attributs d'approbation des médicaments ciblant les protéines en fonction des informations de protéine stockées dans l'unité de stockage de cible de médicament (106c), qui sont les protéines que le réseau de similarité de protéine inclut, obtenir pendant le développement de médicament un résultat de détermination représentant si les protéines à valider, qui sont des protéines que le réseau de similarité inclut, se situent dans une plage de cibles de médicaments approuvés ou une plage de cibles de médicaments rejetés, en utilisant les mesures de centralité de similarité des protéines à valider qui sont calculées à l'étape de calcul de mesure de centralité de similarité (SA-1) ; et
une étape de sortie de résultat de détermination (SA-3) consistant à sortir, via l'unité de sortie, le résultat de détermination qui est obtenu à l'étape de détermination d'approbation (SA-2).

7. Produit de programme informatique ayant un support lisible par ordinateur tangible non transitoire incluant des instructions programmées pour amener, lorsqu'elles sont exécutées par un appareil de prédiction d'approbation (100) incluant une unité de sortie, une unité de stockage (106), et une unité de commande (102), dans lequel l'unité de stockage (106) inclut :

une unité de stockage d'informations de réseau de similarité (106b) configurée pour stocker des informations de réseau de similarité sur un réseau de similarité de protéine qui est construit en fonction de la similarité entre protéines, ladite similarité entre protéines étant trouvée avec un algorithme basé sur une signature de protéine déterminant la similarité entre deux informations de séquence, lesdites informations de réseau de similarité étant déterminées comme un graphe comprenant :

- des nœuds, chaque nœud représentant une protéine, et
- des arêtes, de sorte que deux nœuds ne soient reliés par une arête que si les nœuds partagent une similarité de séquence de protéine considérable ;

une unité de stockage de cible de médicament (106c) configurée pour stocker des informations de médicament contenant des attributs d'approbation de médicaments sur l'approbation ou le rejet et des informations de protéine sur les protéines ciblées par les médicaments en association les unes avec les autres ; et

une unité de stockage d'informations de réseau d'interaction (106d) configurée pour stocker des informations de réseau d'interaction sur un réseau d'interaction protéine-protéine qui est construit sur la base d'interactions entre des protéines prédéterminées, ladite interaction entre protéines signifiant que lesdites protéines sont similaires, lesdites informations de réseau d'interaction étant déterminées comme un graphe comprenant :

- des nœuds, chaque nœud représentant une protéine, et
- des arêtes de sorte que deux nœuds soient reliés si les protéines correspondantes ont une interaction ;

l'appareil de prédiction d'approbation (100) à effectuer un procédé de prédiction d'approbation comprenant :

une étape de calcul de mesure de centralité de similarité (SB-1) consistant à, sur la base des informations de réseau de similarité stockées dans l'unité de stockage d'informations de réseau de similarité (106b), calculer des mesures de centralité de similarité qui sont des mesures de centralité contenant :

- une centralité de degré, définie comme un indice représentant le degré auquel le nœud est relié directement à des nœuds dans le réseau de similarité,
- une centralité d'intermédiarité B(v), utilisant l'expression (1) suivante formée de $S_{ij}$ désignant le nombre de chemins les plus courts entre un nœud i et un nœud j, et $S_{ij}(v)$ désignant la fraction de chemins les plus courts traversant un nœud v :

$$(1)\ \mathrm{B(v)} = \sum \frac{S_{ij}(v)}{S_{ij}}\ \text{avec } \mathrm{i} \neq \mathrm{j},\ \mathrm{v} \neq \mathrm{i}\ \text{et } \mathrm{v} \neq \mathrm{j},$$

- une centralité de proximité C(v), utilisant l'expression (2) suivante formée de d(v, i) désignant la distance représentée à l'étape entre un nœud v et le nœud i :

$$(2)\ \mathrm{C(v)} = \frac{1}{\sum d(v,i)}\ \text{avec } \mathrm{i} \neq \mathrm{v},$$

et

- une contrainte de Burt C(i) des protéines que le réseau de similarité de protéine inclut, utilisant l'expression (3) suivante formée de $p_{iq}p_{qj}$ désignant un produit de l'intensité proportionnelle de la relation du nœud j avec le nœud i et de l'intensité proportionnelle de la relation du nœud j avec le nœud q :

$$\mathrm{C(i)} = \sum_{j}\left(p_{ij} + \sum_{q} p_{iq}p_{qj}\right)^2$$
$$(3)\qquad\qquad\qquad \text{avec } \mathrm{q} \neq \mathrm{i},\ \mathrm{j}\ \text{et } \mathrm{j} \neq \mathrm{i} ;$$

une étape de calcul de mesure de centralité d'interaction (SB-2) consistant à, sur la base des informations de réseau d'interaction stockées dans l'unité de stockage d'informations de réseau d'interaction (106d), calculer des mesures de centralité d'interaction qui sont des mesures de centralité contenant lesdites centralité de degré, centralité d'intermédiarité calculée avec l'expression (1), centralité de proximité calculée avec l'expression (2), et contrainte de Burt des protéines que le réseau d'interaction protéine-protéine inclut calculée avec l'expression (3) ;

une étape de calcul de score de rejet (SB-3) consistant à calculer pendant le développement de médicament un score de rejet qui représente une probabilité qu'un composé à valider soit classifié comme un médicament rejeté, en utilisant des classificateurs qui utilisent, en tant que données d'apprentissage, les attributs d'approbation des médicaments respectifs stockés dans l'unité de stockage de cible de médicament (106c), la somme et la moyenne des mesures de centralité de similarité par cible pour chaque médicament qui sont calculées à l'étape de calcul de mesure de centralité de similarité (SB-1), et la somme et la moyenne des mesures de centralité d'interaction par cible pour chaque médicament qui sont calculées à l'étape de calcul de mesure de centralité d'interaction (SB-2), lesdites mesures de centralité de similarité utilisées en tant que paramètres pour plusieurs classificateurs d'apprentissage machine ; et

une étape de sortie de score de rejet (SB-4) consistant à sortir, via l'unité de sortie, le score de rejet qui est calculé à l'étape de calcul de score de rejet (SB-3).

**8.** Produit de programme informatique ayant un support lisible par ordinateur tangible non transitoire incluant des instructions programmées pour amener, lorsqu'elles sont exécutées par un appareil de prédiction d'approbation (100) incluant une unité de sortie, une unité de stockage (106), et une unité de commande (102), dans lequel l'unité de stockage (106) inclut :

une unité de stockage d'informations de réseau de similarité (106b) configurée pour stocker des informations de réseau de similarité sur un réseau de similarité de protéine qui inclut des protéines ayant une similarité, ladite similarité entre protéines étant trouvée avec un algorithme basé sur une signature de protéine déterminant la similarité entre deux informations de séquence, lesdites informations de réseau de similarité étant déterminées comme un graphe comprenant :

- des nœuds, chaque nœud représentant une protéine, et
- des arêtes, de sorte que deux nœuds ne soient reliés par une arête que si les nœuds partagent une similarité de séquence de protéine considérable ; et

une unité de stockage de cible de médicament (106c) configurée pour stocker des informations de médicament contenant des attributs d'approbation de médicaments sur l'approbation ou le rejet et des informations de protéine sur les protéines ciblées par les médicaments en association les unes avec les autres ;

l'appareil de prédiction d'approbation (100) à effectuer un procédé de prédiction d'approbation comprenant :

une étape de calcul de mesure de centralité de similarité (SA-1) consistant à, sur la base des informations de réseau de similarité stockées dans l'unité de stockage d'informations de réseau de similarité (106b), calculer des mesures de centralité de similarité qui sont des mesures de centralité contenant :

- une centralité de degré, définie comme un indice représentant le degré auquel le nœud est relié directement à des nœuds dans le réseau de similarité,
- une centralité d'intermédiarité B(v), utilisant l'expression (1) suivante formée de $S_{ij}$ désignant le nombre de chemins les plus courts entre un nœud i et un nœud j, et $S_{ij}(v)$ désignant la fraction de chemins les plus courts traversant un nœud v :

$$(1)\ \mathrm{B(v)} = \sum \frac{S_{ij}(v)}{S_{ij}} \text{ avec } i \neq j,\ v \neq i \text{ et } v \neq j,$$

- une centralité de proximité C(v), utilisant l'expression (2) suivante formée de d(v, i) désignant la distance représentée à l'étape entre un nœud v et le nœud i :

$$(2)\ \mathrm{C(v)} = \frac{1}{\sum d(v,i)} \text{ avec } i \neq v,$$

et
- une contrainte de Burt C(i) des protéines que le réseau de similarité de protéine inclut, utilisant l'expression (3) suivante formée de $p_{iq}p_{qj}$ désignant un produit de l'intensité proportionnelle de la relation du nœud j avec le nœud i et de l'intensité proportionnelle de la relation du nœud j avec le nœud q :

$$C(i) = \sum_j (p_{ij} + \sum_q p_{iq} + p_{qj})^2$$

(3) $\qquad\qquad\qquad\qquad$ avec $q \neq i$, j et $j \neq i$ ;

une étape de détermination d'approbation (SA-2) consistant à, sur la base des attributs d'approbation des médicaments ciblant les protéines en fonction des informations de protéine stockées dans l'unité de stockage de cible de médicament (106c), qui sont les protéines que le réseau de similarité de protéine inclut, obtenir pendant le développement de médicament un résultat de détermination représentant si les protéines à valider, qui sont des protéines que le réseau de similarité inclut, se situent dans une plage de cibles de médicaments approuvés ou une plage de cibles de médicaments rejetés, en utilisant les mesures de centralité de similarité des protéines à valider qui sont calculées à l'étape de calcul de mesure de centralité de similarité (SA-1) ; et

une étape de sortie de résultat de détermination (SA-3) consistant à sortir, via l'unité de sortie, le résultat de détermination qui est obtenu à l'étape de détermination d'approbation (SA-2).

# FIG.1

```
        ┌─────────────┐
        │    START    │
        └─────────────┘
               │
               ▼
   ┌───────────────────────┐
   │  CALCULATE SIMILARITY  │──── SA-1
   │   CENTRALITY MEASURE   │
   └───────────────────────┘
               │
               ▼
   ┌───────────────────────┐
   │ OBTAIN DETERMINATION   │──── SA-2
   │        RESULT          │
   └───────────────────────┘
               │
               ▼
   ┌───────────────────────┐
   │ OUTPUT DETERMINATION   │──── SA-3
   │        RESULT          │
   └───────────────────────┘
               │
               ▼
        ┌─────────────┐
        │     END     │
        └─────────────┘
```

# FIG.2

```
        ┌─────────────┐
        │    START    │
        └─────────────┘
               │
               ▼
   ┌───────────────────────┐
   │  CALCULATE SIMILARITY  │──── SB-1
   │   CENTRALITY MEASURE   │
   └───────────────────────┘
               │
               ▼
   ┌───────────────────────┐
   │  CALCULATE INTERACTION │──── SB-2
   │   CENTRALITY MEASURE   │
   └───────────────────────┘
               │
               ▼
   ┌───────────────────────┐
   │ CALCULATE REJECTION    │──── SB-3
   │        SCORE           │
   └───────────────────────┘
               │
               ▼
   ┌───────────────────────┐
   │  OUTPUT REJECTION      │──── SB-4
   │        SCORE           │
   └───────────────────────┘
               │
               ▼
        ┌─────────────┐
        │     END     │
        └─────────────┘
```

# FIG.3

# FIG.4

PROTEIN
SIGNATURE-
BASED
ALGORITHM

ALGORITHM
FOR
CALCULATING
CENTRALITY
REFERENCE

106b

HUMAN
PROTEIN
DATABASE

106a

SC-1

PROTEIN
SIMILARITY
NETWORK

SC-2

Prot A -> 0.054 0.540
Prot B -> 0.540 0.344
Prot C -> 0.454 0.323
Prot D -> 0.234 0.325
Prot E -> 0.323 0.354
Prot F -> 0.744 0.237
Prot G -> 0.478 0.584

SC-3

SC-4

SAFENESS OF
TARGET
PROTEIN

106c

DRUG TARGET
DATABASE

MACHINE
LEARNING
CLASSIFIER

106d

PROTEIN-PROTEIN
INTERACTION
NETWORK

SC-5

SC-6

REJECTION
SCORE OF
COMPOUND

SC-7

EP 2 905 363 B1

37

# FIG.5

>**P63261**

MEEEIAALVIDNGSGMCKAGFAGDDAPRAVFPSIVGRPRHQGVMVGMGQKDSYVGDEAQS
KRGILTLKYPIEHGIVTNWDDMEKIWHHTFYNELRVAPEEHPVLLTEAPLNPKANREKMT
QIMFETFNTPAMYVAIQAVLSLYASGRTTGIVMDSGDGVTHTVPIYEGYALPHAILRLDL
AGRDLTDYLMKILTERGYSFTTTAEREIVRDIKEKLCYVALDFEQEMATAASSSSLEKSY
ELPDGQVITIGNERFRCPEALFQPSFLGMESCGIHETTFNSIMKCDVDIRKDLYANTVLS
GGTTMYPGIADRMQKEITALAPSTMKIKIIAPPERKYSVWIGGSILASLSTFQQMWISKQ
EYDESGPSIVHRKCF

>**P49281**

MVLGPEQKMSDDSVSGDHGESASLGNINPAYSNPSLSQSPGDSEEYFATYFNEKISIPEE
EYSCFSFRKLWAFTGPGFLMSIAYLDPGNIESDLQSGAVAGFKLLWILLLATLVGLLLQR
LAARLGVVTGLHLAEVCHRQYPKVPRVILWLMVELAIIGSDMQEVIGSAIAINLLSVGRI
PLWGGVLITIADTFVFLFLDKYGLRKLEAFFGFLITIMALTFGYEYVTVKPSQSQVLKGM
FVPSCSGCRTPQIEQAVGIVGAVIMPHNMYLHSALVKSRQVNRNNKQEVREANKYFFIES
CIALFVSFIINVFVVSVFAEAFFGKTNEQVVEVCTNTSSPHAGLFPKDNSTLAVDIYKGG
VVLGCYFGPAALYIWAVGILAAGQSSTMTGTYSGQFVMEGFLNLKWSRFARVVLTRSIAI
IPTLLVAVFQDVEHLTGMNDFLNVLQSLQLPFALIPILTFTSLRPVMSDFANGLGWRIAG
GILVLIICSINMYFVVVYVRDLGHVALYVVAAVVSVAYLGFVFYLGWQCLIALGMSFLDC
GHTCHLGLTAQPELYLLNTMDADSLVSR

# FIG.6

Q3MI94 Q9Y473 210.901 536 1.67035e-54 82 119 1 214 377
GKMFGQNSTLVIHKAIHTGEKPYKCNECGKAFNQQSHLSRHHRLHTGEKPYKCNDCGKAFIHQSSLARHHRLHTGEKSYKCEECDRVFS
QKSNLERHKIIHTGEKPYKCNECHKTFSHRSSLPCHRRLHSGEKPYKCNECGKTFNVQSHLSRHHRLHTGEKPYKCKVCDKAFMCHSYLA
N H T R I H S G E K P Y K C N E C G K A H N H L I D S S I K P C M S S 590 230
GQLFSHSSSDACSKNIHTGETFCKGNQCRKVCGHKQSLKQHQ-IHTQKKPDGCSECGGSFTQKSHLFAQQRIHSVGNLHECGKCGKAFM
PQLKLSVYLTDHTGDIPCICKECGKVFIQRSELLTHQKTHTRKKPYKCHDCGKAFFQMLSLFRHQRTHSREKLYECSECGKGFSQNSTLIIH
QKIHTGERQYACSECGKAFTQKSTLSLHQRIHS 442 G++F +S+ K IHTGE K N+C K + L +H +HT +KP C++CG +F
+S L R+H+ ++C +C + F + L + HTG+ P C E C K F RS L H++ H+ +KPYKC++CGK F L RH R H+ EK Y+C C K F
+S L H +IH+GE+ Y C+ECGKA S+ + S

Q9P2V4 Q8N0V4 94.6147 232 1.58178e-19 47 82 23 291 23
CPSQCSCSLHIMGDGSKARTVVCNDPDMTLPPASIPPDTSRLRLERTAIRRVPGEAFRPLGRLEQLWLPYNALSELNALMLRGLRRLRELR
LPGNRLAAFPWAALRDAPKLRLLDLQANRLSAVPAEAARFLENLTFLDLSSNQLMRLPQELIVSWAHLETGIFPPGHHPRRVLGLQDNPWA
CDCRLYDLVHLLDGWAPNLAFIETELRCASPRSLAGVAFSQLELRKCQGPELHPGVASIRSLLGGTALLRCGATGVPGPEMSWRRANGRPL
N G T V H Q E V S S D G T S W T L L 313 3 6
CPATCSCTKESIICVGS-----------SWVPRIVPGDISSLSLVNGTFSEIKDRMFSHLPSLQLLLLNSNSFTIIRDDAFAGLFHLEYLFIEGNKIETISR
NAFRGLRDLTHLSLANNHIKALPRDVFSDLDSLIELDLRGNKFECDCKAKWLYLWLKMTNS------------TVSDVLCIGPPEYQEKKLNDVTSFD
YECTTTDFVVHQTLPYQSVSVDTFNSKNDVYVAIAQPSMENCMVLEWDHIEMNFRSYDNITGQSIVGCKAILIDDQVFVVVAQLFGGSHIY
303 CP+ CSC+ + + P +PD S L L + F L L+ L L N+ + + GL L L + GN++
A R L L L N + A+P + L++L LDL N+ + + T + Y L D + + T+ V+ +
+ +L + + S ++ V V+ +

EP 2 905 363 B1

# FIG.7

# FIG.8

| Protein ID | Degree | Betweenness | Closeness Centrality | Burts Constraint |
|---|---|---|---|---|
| P14784 | 55 | 12671.535 | 0.000 | 0.043 |
| P14854 | 1 | 0.000 | 5.0709939148073e-05 | 1.000 |
| P14859 | 224 | 544.657 | 0.000 | 0.016 |
| P14867 | 59 | 0.000 | 0.000 | 0.066 |
| P14868 | 4 | 0.000 | 5.07176548156413e-05 | 0.766 |
| P14902 | 1 | 0.000 | 5.0709939148073e-05 | 1.000 |
| P14920 | 1 | 0.000 | 5.0709939148073e-05 | 1.000 |

# FIG.9

| Drug | Targets | Status |
|---|---|---|
| Acetylsalicylic acid | P23219 | Approved |
| Glycodiazine | P48048, Q09428 | Approved |
| Phenacetin | P23219 | Withdraw |

# FIG.10

EP 2 905 363 B1

# FIG.11

| Protein 1 | Protein 2 |
|-----------|-----------|
| AL1A1_HUMAN | AL1A1_HUMAN |
| ITA7_HUMAN | ACHA_HUMAN |
| NEB1_HUMAN | ACTG_HUMAN |
| SRGN_HUMAN | CD44_HUMAN |
| GRB7_HUMAN | ERBB2_HUMAN |
| PAK1_HUMAN | ERBB2_HUMAN |
| DLG4_HUMAN | ERBB2_HUMAN |
| P85B_HUMAN | ERBB2_HUMAN |
| PTN18_HUMAN | ERBB2_HUMAN |

# FIG.12

# FIG.13

# FIG.14

| Classifier | Reference |
| --- | --- |
| ZeroR | (Witten, et al., 2011) |
| Naive Bayes | (John and Langley, 1995) |
| Threshold Selector | (Witten, et al., 2011) |
| MLP | (Cybenko, 1992) |
| JRip | (Cohen, 1995) |
| IB1 | (Aha, et al., 1991) |
| PART | (Frank and Witten, 1998) |
| END | (Dong, et al., 2005) |
| Class Balanced ND | (Dong, et al., 2005) |
| IBk | (Aha, et al., 1991) |
| Random Tree | (Witten, et al., 2011) |
| Rotation Forest | (Rodriguez and Kuncheva, 2006) |
| Random Forest | (Breiman, 2001) |
| Decorate | (Melville and Mooney, 2003) |
| KStar | (Cleary and Trigg, 1995) |

# FIG.15

| Drug | True Class | Algorithm 1 | Algorithm 2 | Algorithm 3 | Algorithm 4 | Rejection Score |
| --- | --- | --- | --- | --- | --- | --- |
| AZD8075 | Problematic | 1.00 | 1.00 | 1.00 | 1.00 | 0.98 |
| Zomepirac | Problematic | 1.00 | 1.00 | 1.00 | 1.00 | 0.98 |
| BMS 275291 | Problematic | 1.00 | 1.00 | 0.99 | 1.00 | 0.98 |
| Yohimbine | Approved | 0.00 | 0.00 | 0.00 | 0.01 | 0.01 |
| Doxepin | Approved | 0.00 | 0.00 | 0.00 | 0.01 | 0.00 |
| Rifabutin | Approved | 0.00 | 0.00 | 0.00 | 0.00 | 0.00 |

## REFERENCES CITED IN THE DESCRIPTION

**Non-patent literature cited in the description**

- **ARMBRUSTER BN ; ERNSBERGER P ; IRWIN JJ ; SHOICHET BK.** Relating protein pharmacology by ligand chemistry. *Nature Biotechnology,* 2007, vol. 25, 197-206
- **CAMPILLOS M ; KUHN M ; GAVIN AC ; JENSEN LJ ; BORK P.** Drug Target Identification Using Side-Effect Similarity. *Science,* 2008, vol. 321, 263-266
- **KEISER MJ ; SETOLA V ; IRWIN JJ ; LAGGNER C ; ABBAS AI ; HUFEISEN SJ ; JENSEN NH ; KUIJER MB ; MATOS RC ; TRAN TB.** Predicting new molecular targets for known drugs. *Nature,* 2009, vol. 462, 175-181
- **YAMANISHI Y ; ARAKI M ; GUTTERIDGE A ; HONDA W ; KANEHISA M.** Prediction of drug target interaction networks from the integration of chemical and genomic spaces. *Bioinformatics,* 2008, vol. 24, i232-i240
- **LOUNKINE E ; KEISER MJ ; WHITEBREAD S ; MIKHAILOV D ; HAMON J ; JENKINS JL ; LAVAN P ; WEBER E ; DOAK AK ; COTE S.** Large-scale prediction and testing of drug activity on side-effect targets. *Nature,* 2012, vol. 486, 361-367
- **LIU Z ; SHI Q ; DING D ; KELLY R ; FANG H et al.** Translating Clinical Findings into Knowledge in Drug Safety Evaluation - Drug Induced Liver Injury Prediction System (DILIps). *PLoS Comput Biol,* 2011, vol. 7 (12), e1002310